# FASCICULE DE BREVET EUROPEEN

(11) **EP 3 714 071 B1**
(45) Date de publication et mention de la délivrance du brevet: **08.01.2025**
(21) Numéro de dépôt: 18821723.6
(22) Date de dépôt: 23.11.2018
(51) Int. Cl.: C12Q 1/6895

(54) **AMORCES UNIVERSELLES ET LEUR UTILISATION POUR LA DÉTECTION ET/OU L'IDENTIFICATION D'ALGUES**
UNIVERSALE PRIMER UND DEREN VERWENDUNG ZUR DETEKTION UND/ODER IDENTIFIZIERUNG VON ALGEN
UNIVERSAL PRIMERS AND THEIR USE FOR DETECTING AND/OR IDENTIFYING ALGAE

(30) Priorité: 23.11.2017 FR 1761116
(43) Date de publication de la demande: 30.09.2020
(73) Titulaire: DNA Gensee SAS, 73377 Le Bourget Du Lac Cedex (FR)
(72) Inventeur: DUBRULLE, Nelly, 73490 La Ravoire (FR); ROCCIA, Aymeric, 73000 Chambery (FR); GIRAUD, Nicole, 38920 Crolles (FR)
(74) Mandataire: Grosset-Fournier, Chantal Catherine
(86) Numéro de dépôt international: PCT/FR2018/052969
(87) Numéro de publication internationale: WO 2019/102162

(56) Documents cités:
- CN-A- 101 153 337
- CN-A- 107 201 405
- US-A- 5 776 680
- GARY W. SAUNDERS ET AL: "Acquiring DNA sequence data from dried archival red algae (Florideophyceae) for the purpose of applying available names to contemporary genetic species: a critical assessment", BOTANY = BOTANIQUE, vol. 90, no. 3, 1 March 2012 (2012-03-01), pages 191 - 203, XP055551616, ISSN: 1916-2790, DOI: 10.1139/b11-079
- ABDELGHANI BEN ALI ET AL: "PHYLOGENETIC RELATIONSHIPS AMONG ALGAE BASED ON COMPLETE LARGE-SUBUNIT RRNA SEQUENCES", INTERNATIONAL JOURNAL OF SYSTEMATIC AND EVOLUTIONARY MICROBIOLOGY, SOCIETY FOR GENERAL MICROBIOLOGY, GB, vol. 51, no. PART 03, 1 May 2001 (2001-05-01), pages 737 - 749, XP008026066, ISSN: 1466-5026
- ABDELGHANI BEN ALI ET AL: "EVOLUTIONARY RELATIONSHIPS AMONG HETEROKONT ALGAE (THE AUTOTROPHIC STRAMENOPILES) BASED ON COMBINED ANALYSES OF SMALL AND LARGE SUBUNIT RIBOSOMAL RNA", PRO, FISCHER, JENA, DE, vol. 153, no. 2, 29 May 2002 (2002-05-29), pages 123 - 132, XP008026263, ISSN: 1434-4610, DOI: 10.1078/1434-4610-00091
- KIMBERLY Y CONKLIN ET AL: "A molecular method for identification of the morphologically plastic invasive algal genera Eucheuma and Kappaphycus (Rhodophyta, Gigartinales) in Hawaii", JOURNAL OF APPLIED PHYCOLOGY, KLUWER ACADEMIC PUBLISHERS, DO, vol. 21, no. 6, 31 January 2009 (2009-01-31), pages 691 - 699, XP019749645, ISSN: 1573-5176, DOI: 10.1007/S10811-009-9404-2
- FREDERIK LELIAERT ET AL: "Phylogeny of the Cladophorophyceae (Chlorophyta) inferred from partial LSU rRNA gene sequences: is the recognition of a separate order Siphonocladales justified?", EUROPEAN JOURNAL OF PHYCOLOGY, vol. 38, no. 3, 1 August 2003 (2003-08-01), DE, pages 233 - 246, XP055482326, ISSN: 0967-0262, DOI: 10.1080/1364253031000136376
- HART ET AL: "Large subunit ribosomal RNA gene variation and sequence heterogeneity of Dinophysis (Dinophyceae) species from Scottish coastal waters", HARMFUL A, ELSEVIER, AMSTERDAM, NL, vol. 6, no. 2, 24 January 2007 (2007-01-24), pages 271 - 287, XP005857797, ISSN: 1568-9883, DOI: 10.1016/J.HAL.2006.10.001

## Description

La présente demande décrit de nouvelles amorces universelles, ainsi que leur utilisation pour la détection et/ou l'identification d'algues.

Le « DNA barcoding » est une méthode de biologie moléculaire permettant de déterminer les espèces présentes dans un échantillon grâce à leur signature génétique, unique pour chaque espèce. L'idée est d'avoir un fragment d'ADN présent chez toutes les espèces à analyser et qui constitue un marqueur génétique. Ce marqueur est un fragment d'ADN encadré par des régions très conservées et donc universelles, et qui, une fois séquencé, montre des variations de séquences génétiques entre individus d'espèces différentes et des séquences identiques entre individus d'une même espèce (HEBERT, Paul DN., CYWINSKA, Alina, BALL, Shelley L. et DEWAARD, Jeremy R. Biological identifications through DNA barcodes. Proceedings of the Royal Society of London B: Biological Sciences, 2003, vol. 270, no. 1512, p. 313-321).

La délimitation des espèces, grâce au « DNA barcoding », est basée sur le ratio « divergence intraspécifique/divergence interspécifique » (HEBERT, Paul DN, STOECKLE, Mark Y., ZEMLAK, Tyler S. et FRANCIS, Charles M. Identification of birds through DNA barcodes. PLoS Biology, 2004, vol. 2, no. 10, p. e312.). Ce ratio est appelé le « barcoding gap ».

Le gène mitochondrial codant pour la première sous-unité de la cytochrome c oxydase (*coxl*) est un exemple de marqueur génétique pouvant être utilisé chez les animaux.

Chez les plantes, les analyses se font à l'aide de plusieurs marqueurs nucléaires et chloroplastiques (tels que le marqueur *trnL,* voir le brevet EP1797201).

De façon similaire aux plantes (CBOL Plant Working Group. A DNA barcode for land plants. PNAS, 2009, vol. 106, no. 31, pp. 12794-12797), l'utilisation d'un seul marqueur génétique universel pour la détermination et/ou l'identification des espèces d'algues est impossible du fait de leurs multiples origines (LELIAERT, Frederik, VERBRUGGEN, Heroen, VANORMELINGEN, Pieter, STEEN, Frédérique, LÔPEZ-BAUTISTA, Juan M., ZUCCARELLO Giuseppe C. et DE CLERCK Olivier. DNA-based species délimitation in algae. European Journal of Phycology, 2014, vol. 49, no. 2, pp. 179-196).

Plusieurs études ont montré qu'il existait des régions très conservées chez les algues. Celles-ci ont suscité un grand intérêt, notamment UPA (Universal Plastid Amplicon). Sa très grande conservation a permis de l'amplifier chez de nombreuses espèces mais il a été considéré que sa séquence manquait de variabilité pour y identifier un marqueur permettant une discrimination au niveau des espèces. D'autres régions conservées ont ainsi été étudiées : ITS (Internai Transcribed Spacer), LSU (Large SubUnit), SSU (Small Subunit), rbcL (ribulose biphosphate carboxylase large chain), tufA, ou encore COX1 (cytochrome oxydase 1). Cependant, les marqueurs identifiés à partir de ces régions manquaient de résolution, notamment pour discriminer des espèces très proches (MATTIO, Lydiane et PAYRI, Claude. Assessment of five markers as potential barcodes for identifying *Sargassum subgenus Sargassum species* (*Phaeophyceae, Fucales*)*.* Cryptogamie Algologie, 2010, vol. 31, no. 4, p. 467.).

On connaît également le document ABDELGHANI BEN ALI ET AL, "PHYLOGENETIC RELATIONSHIPS AMONG ALGAE BASED ON COMPLETE LARGE-SUBUNIT RRNA SEQUENCES", INTERNATIONAL JOURNAL OF SYSTEMATIC AND EVOLUTIONARY MICROBIOLOGY, SOCIETY FOR GENERAL MICROBIOLOGY, GB, (20010501), vol. 51, no. PART 03 qui décrit l'étude des relations phylogénétiques de plusieurs groupes d'algues tels que les chlorarachniophytes, les cryptomonades, les haptophytes, les bacillariophytes, les dictyochophytes et les pélagophytes en utilisant de façon combinée les séquences d'ARNr de la sous-unité large (LSU) et de la petite sous-unité (SSU) pour la détection et l'identification desdites algues, le document ABDELGHANI BEN ALI ET AL, "EVOLUTIONARY RELATIONSHIPS AMONG HETEROKONT ALGAE (THE AUTOTROPHIC STRAMENOPILES) BASED ON COMBINED ANALYSES OF SMALL AND LARGE SUBUNIT RIBOSOMAL RNA", PRO, FISCHER, JENA, DE, (20020529), vol. 153, no. 2 pages 123 - 132, qui décrit l'étude des relations phylogénétiques chez les straménopiles, et en particulier chez les hétérokontes. Le document FREDERIK LELIAERT ET AL, "Phylogeny of the Cladophorophyceae (Chlorophyta) inferred from partial LSU rRNA gène séquences: is the récognition of a separate order Siphonocladales justified?", EUROPEAN JOURNAL OF PHYCOLOGY, DE, (20030801), vol. 38, no. 3, qui décrit les relations phylogénétiques au sein de la classe des algues vertes Cladophorophyceae et le document HART ET AL, "Large subunit ribosomal RNA gène variation and séquence heterogeneity of Dinophysis (Dinophyceae) species from Scottish coastal waters", HARMFUL A, ELSEVIER, AMSTERDAM, NL, (20070124), vol. 6, no. 2, qui décrit une étude visant la diversité génétique des espèces de *Dinophysis* autour de la côte écossaise. Des amorces eucaryotes universelles ont été utilisées pour amplifier le gène de l'ARN ribosomal (ARNr) de la grande sous-unité (LSU). Les marqueurs de détection et/ou d'identification mentionnés dans ces documents ont une taille très supérieure à 220 nucléotides.

Les marqueurs pouvant être utilisés communément pour la détermination et/ou l'identification des espèces d'algues restent encore à identifier. En effet, il n'existe pas, à ce jour, de marqueur suffisamment variable et possédant des amorces universelles pour permettre une bonne résolution par le « DNA barcoding » chez les algues. Ceci est d'autant plus vrai pour l'analyse par le « DNA barcoding de substrats dégradés, c'est-à-dire des produits dans lesquels l'ADN d'algue est dégradé et dont la taille est inférieure ou égale à 220 paires de bases.

La détermination et/ou l'identification d'algues à partir d'ADN dégradé est très importante, notamment à des fins de contrôle-qualité de produits alimentaires, de compléments alimentaires ou cosmétiques pour lesquels l'utilisation d'algues connaît un succès croissant. Néanmoins, les régions d'ADN d'algue suffisamment courtes (pour pouvoir analyser des échantillons d'ADN dégradé) et suffisamment variables (pour permettre de déterminer et/ou d'identifier les algues, notamment les espèces d'algues) sont rares et à ce jour aucune n'a été caractérisée. De plus, il n'existe pas d'amorces universelles encadrant un tel fragment d'ADN d'algue (de taille inférieure ou égale à 220 paires de bases) permettant d'amplifier de l'ADN d'algue dégradé.

Il existe ainsi un réel de besoin de pouvoir déterminer et/ou identifier les algues à partir d'ADN d'algue dégradé.

Il existe également un réel besoin de déterminer des régions d'ADN d'algue suffisamment courtes et suffisamment variables pour pouvoir être utilisées dans la détermination et/ou l'identification d'algue.

Il existe aussi un réel besoin de déterminer des amorces universelles permettant d'amplifier lesdites régions d'ADN d'algue suffisamment courtes et suffisamment variables, notamment de nouvelles amorces universelles permettant d'amplifier de l'ADN d'algue dégradé (de taille inférieure ou égale à 220 paires de bases).

Il existe donc un réel besoin de déterminer un marqueur suffisamment variable et possédant des amorces universelles pour permettre une bonne résolution par le « DNA barcoding » chez les algues.

C'est pourquoi l'un des buts de la présente demande est de fournir des régions d'ADN d'algue suffisamment courtes et suffisamment variables pour pouvoir être utilisées dans la détermination et/ou l'identification d'algues.

L'un des buts de la présente demande est également de fournir des amorces universelles permettant d'amplifier lesdites régions d'ADN d'algue suffisamment courtes et suffisamment variables.

L'un des buts de la présente demande est ainsi de fournir un procédé de détermination et/ou d'identification d'algue à partir d'ADN d'algue dégradé.

La présente demande repose en effet sur la détermination, par les Inventeurs, (i) de régions d'ADN d'algue suffisamment courtes et suffisamment variables pour pouvoir être utilisées dans la détermination et/ou l'identification d'algues, et (ii) des amorces universelles permettant d'amplifier lesdites régions d'ADN d'algue. Ces courtes régions d'ADN d'algue constituent des marqueurs dits « courts » et ont été identifiées dans les régions LSU des algues.

La présente invention est exposée dans le jeu de revendications joint et a pour premier objet l'utilisation d'une paire d'amorces d'amplification pour la détection et/ou l'identification d'une algue dans un échantillon susceptible d'en contenir, par amplification d'au moins une région variable du gène *LSU* codant pour la grande sous-unité d'un ribosome, dans laquelle ladite région variable amplifiée du gène LSU d'une algue a une taille inférieure ou égale à 220 paires de bases, ladite région variable constituant un marqueur de détection et/ou d'identification d'une algue, dans laquelle:
- une première paire d'amorces d'amplification consiste en un premier oligonucléotide qui s'hybride à une séquence de 15 à 30 nucléotides contigus, de préférence de 18 à 24 nucléotides contigus, choisie dans la séquence de SEQ ID NO: 1 du gène *LSU* d'une algue et un deuxième oligonucléotide qui s'hybride à une séquence de 15 à 30 nucléotides contigus, de préférence de 18 à 24 nucléotides contigus, choisie dans la séquence de SEQ ID NO: 2 du gène *LSU* d'une algue, ou
- une deuxième paire d'amorces d'amplification consiste en un premier oligonucléotide qui s'hybride à une séquence de 15 à 30 nucléotides contigus, de préférence de 18 à 24 nucléotides contigus choisie dans la séquence de SEQ ID NO: 5 du gène *LSU* d'une algue et le deuxième oligonucléotide s'hybride à une séquence de 15 à 30 nucléotides contigus, de préférence de 18 à 24 nucléotides contigus choisie dans la séquence de SEQ ID NO: 6 du gène *LSU* d'une algue.

Un second objet de la présente invention concerne une paire d'amorces d'amplification ayant une taille comprise de 15 à 45 nucléotides, notamment de 15 à 30 nucléotides contigus, de préférence de 18 à 24 nucléotides contigus, dans laquelle une première paire d'amorces d'amplification comprend ou est constituée par
- SEQ ID NO: 1, ou une séquence ayant un pourcentage d'identité d'au moins 80%, 81%, 82%, 83%, 84%, 85%, 86%, 86%, 88%, 89%, 90%, 91%, 92%, 93%, 94%, 95%, 96%, 97%, 98%, 99% avec ladite séquence de SEQ ID NO: 1, et
- SEQ ID NO: 2, ou une séquence ayant un pourcentage d'identité d'au moins 80%, 81%, 82%, 83%, 84%, 85%, 86%, 86%, 88%, 89%, 90%, 91%, 92%, 93%, 94%, 95%, 96%, 97%, 98%, 99% avec ladite séquence de SEQ ID NO: 2, dans laquelle une seconde paire d'amorces d'amplification comprend ou est constituée par
- SEQ ID NO: 5, ou une séquence ayant un pourcentage d'identité d'au moins 80%, 81%, 82%, 83%, 84%, 85%, 86%, 86%, 88%, 89%, 90%, 91%, 92%, 93%, 94%, 95%, 96%, 97%, 98%, 99% avec ladite séquence de SEQ ID NO: 5, et
- SEQ ID NO: 6, ou une séquence ayant un pourcentage d'identité d'au moins 80%, 81%, 82%, 83%, 84%, 85%, 86%, 86%, 88%, 89%, 90%, 91%, 92%, 93%, 94%, 95%, 96%, 97%, 98%, 99% avec ladite séquence de SEQ ID NO: 6, permettant d'amplifier un fragment d'une taille inférieure ou égale à 220 paires de bases d'une région variable du gène LSU codant pour la grande sous-unité d'un ribosome, d'une algue.

Un troisième objet de la présente invention concerne un marqueur de détection et/ou d'identification d'une algue ayant une taille inférieure ou égale à 220 nucléotides d'une région variable amplifiée du gène LSU codant pour la grande sous-unité d'un ribosome, d'une algue , notamment inférieure ou égale à 190, 180 et notamment 178 nucléotides, ledit marqueur comprenant ou étant constitué par une séquence ayant un pourcentage d'identité d'au moins 98%, 99%, ou 100% avec une séquence choisie parmi, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, et SEQ ID NO: 19.

Un dernier objet de la présente invention concerne un procédé d'amplification d'au moins une région variable du gène *LSU* d'une algue, ladite région variable amplifiée du gène LSU codant pour la grande sous-unité d'un ribosome d'une taille inférieure ou égale à 220 paires de bases constituant un marqueur de détection et/ou d'identification d'une algue, comprenant ou étant constitué des étapes suivantes:
a) une étape d'extraction du matériel nucléique d'un produit ou échantillon susceptible de contenir une algue,
b) une étape d'amplification de ladite région variable du gène *LSU* d'une algue, en utilisant
   - une première paire d'amorces d'amplification consiste en un premier oligonucléotide qui s'hybride à une séquence de 15 à 30 nucléotides contigus, de préférence de 18 à 24 nucléotides contigus choisie dans la séquence de SEQ ID NO: 1 du gène *LSU* d'une algue et un deuxième oligonucléotide qui s'hybride à une séquence de 15 à 30 nucléotides contigus, de préférence de 18 à 24 nucléotides contigus choisie dans la séquence de SEQ ID NO: 2 du gène *LSU* d'une algue, ou
   - une deuxième paire d'amorces d'amplification consiste en un premier oligonucléotide qui s'hybride à une séquence de 15 à 30 nucléotides contigus, de préférence de 18 à 24 nucléotides contigus choisie dans la séquence de SEQ ID NO: 5 du gène *LSU* d'une algue et le deuxième oligonucléotide s'hybride à une séquence de 15 à 30 nucléotides contigus, de préférence de 18 à 24 nucléotides contigus choisie dans la séquence de SEQ ID NO: 6 du gène *LSU* d'une algue,
c) une étape de détermination ou d'identification de la présence d'une algue dans ledit produit ou échantillon par la détection d'au moins une région variable amplifiée du gène *LSU* d'une algue.

En outre, la présente demande décrit l'utilisation d'une paire d'amorces d'amplification pour la détection et/ou l'identification d'une algue.

La présente demande décrit aussi les amorces, les séquences cibles desdites amorces, et les régions amplifiées *per se.*

La présente demande décrit aussi l'utilisation des régions amplifiées pour la détermination et/ou l'identification d'une algue.

La présente demande décrit aussi un procédé d'amplification d'au moins une région variable constituant un marqueur de détection et/ou d'identification d'une algue.

La présente demande décrit donc, l'utilisation d'une paire d'oligonucléotides (amorces d'amplification) dans laquelle:
- une première paire d'amorces d'amplification consiste en un premier oligonucléotide qui s'hybride à une séquence de 15 à 30 nucléotides contigus, de préférence de 18 à 24 nucléotides contigus, choisie dans la séquence de SEQ ID NO : 1 du gène *LSU* d'une algue et un deuxième oligonucléotide qui s'hybride à une séquence de 15 à 30 nucléotides contigus, de préférence de 18 à 24 nucléotides contigus, choisie dans la séquence de SEQ ID NO: 2 du gène LSU d'une algue, ou
- une deuxième paire d'amorces d'amplification consiste en un premier oligonucléotide qui s'hybride à une séquence de 15 à 30 nucléotides contigus, de préférence de 18 à 24 nucléotides contigus choisie dans la séquence de SEQ ID NO: 5 du gène *LSU* d'une algue et le deuxième oligonucléotide s'hybride à une séquence de 15 à 30 nucléotides contigus, de préférence de 18
à 24 nucléotides contigus choisie dans la séquence de SEQ ID NO: 6 du gène *LSU* d'une algue, pour la détection et/ou l'identification d'une algue dans un échantillon susceptible d'en contenir, par amplification d'au moins une région variable du gène *LSU* d'une algue, ladite région variable constituant un marqueur de détection et/ou d'identification d'une algue.

Un autre objet décrit de la présente demande est l'utilisation d'une paire d'amorces d'amplification pour la détection et/ou l'identification d'une algue dans un échantillon susceptible d'en contenir, par amplification d'au moins une région variable du gène *LSU* d'une algue d'une taille inférieure à 220 paires de bases, notamment inférieure ou égale à 200, 190, 180 et notamment inférieure ou égale à 178 paires de bases, ladite région variable constituant un marqueur de détection et/ou d'identification d'une algue.

Un objet décrit plus préféré de la présente demande est l'utilisation d'une paire d'amorces d'amplification, dans laquelle:
- une première paire d'amorces d'amplification consiste en un premier oligonucléotide qui s'hybride à une séquence de 15 à 30 nucléotides contigus, de préférence de 18 à 24 nucléotides contigus, choisie dans la séquence de SEQ ID NO: 1 du gène *LSU* d'une algue et un deuxième oligonucléotide qui s'hybride à une séquence de 15 à 30 nucléotides contigus, de préférence de 18 à 24 nucléotides contigus, choisie dans la séquence de SEQ ID NO: 2 du gène *LSU* d'une algue, ou
- une deuxième paire d'amorces d'amplification consiste en un premier oligonucléotide qui s'hybride à une séquence de 15 à 30 nucléotides contigus, de préférence de 18 à 24 nucléotides contigus choisie dans la séquence de SEQ ID NO: 5 du gène *LSU* d'une algue et le deuxième oligonucléotide s'hybride à une séquence de 15 à 30 nucléotides contigus, de préférence de 18 à 24 nucléotides contigus choisie dans la séquence de SEQ ID NO: 6 du gène *LSU* d'une algue.

Le terme « oligonucléotide » signifie une courte séquence de nucléotides, dont le nombre varie d'une dizaine à environ 100 bases (ou 100 nucléotides en considérant la présence d'un ose et d'un, de deux ou de trois groupes phosphate; la relation base/nucléotides étant bien connue de l'homme du métier). Ainsi, l'expression « paire d'oligonucléotides » fait référence à l'association de deux oligonucléotides. Les deux oligonucléotides formant la paire d'oligonucléotides (amorces d'amplification) n'ont pas nécessairement la même taille: par exemple le premier oligonucléotide qui s'hybride à la séquence de SEQ ID NO: 1 peut comprendre 19 nucléotides et le deuxième oligonucléotide qui s'hybride à une séquence de 15 à 30 nucléotides contigus, de préférence de 18 à 24 nucléotides contigus choisie dans la séquence de SEQ ID NO: 2 peut comprendre 20 nucléotides, par exemple.

Les lettres « W », « M » et « R » dans les séquences SEQ ID NO: 41 ou SEQ ID NO: 9 représentent un nucléotide choisi parmi A, T, C ou G.

Dans un aspect particulier de la présente demande, la paire d'oligonucléotides correspond à une paire d'amorces d'amplification. Dans un aspect encore plus particulier de la présente demande, le premier oligonucléotide correspond à une amorce sens et le deuxième oligonucléotide correspond à une amorce anti-sens.

L'expression «une séquence de 15 à 30 nucléotides contigus, de préférence de 18 à 24 nucléotides contigus» signifie 15, 16, 17, 18, 19, 20, 21, 22, 23, 24, 25, 26, 27, 28, 29, 30 nucléotides et de préférence 18, 19, 20, 21, 22, 23, 24 nucléotides.

Le terme «contigus» signifie que les oligonucléotides s'hybrident aux 18 à 24 nucléotides successifs, situés les uns à la suite des autres, sans discontinuité.

Le terme « SEQ ID NO: 1» doit s'entendre comme une séquence de SEQ ID NO : 1 ou une séquence ayant un pourcentage d'identité d'au moins 80%, 81%, 82 %, 83%, 84%, 85%, 86%, 87%, 88%, 89%, 90%, 91%, 92%, 93%, 94%, 95%, 96%, 97%, 98%, 99% ou 100% avec ladite SEQ ID NO : 1, notamment 95% et plus particulièrement 98% ou 99%.

Le terme «SEQ ID NO: 2» doit s'entendre comme une séquence de SEQ ID NO : 2 ou une séquence ayant un pourcentage d'identité d'au moins 80%, 81%, 82 %, 83%, 84%, 85%, 86%, 87%, 88%, 89%, 90%, 91%, 92%, 93%, 94%, 95%, 96%, 97%, 98%, 99% ou 100% avec ladite SEQ ID NO : 2, notamment 95% et plus particulièrement 98% ou 99%.

Le terme «SEQ ID NO: 5» doit s'entendre comme une séquence de SEQ ID NO : 5 ou une séquence ayant un pourcentage d'identité d'au moins 80%, 81%, 82 %, 83%, 84%, 85%, 86%, 87%, 88%, 89%, 90%, 91%, 92%, 93%, 94%, 95%, 96%, 97%, 98%, 99% ou 100% avec ladite SEQ ID NO : 5, notamment 95% et plus particulièrement 98% ou 99%.

Le terme «SEQ ID NO: 6» doit s'entendre comme une séquence de SEQ ID NO : 6 ou une séquence ayant un pourcentage d'identité d'au moins 80%, 81%, 82 %, 83%, 84%, 85%, 86%, 87%, 88%, 89%, 90%, 91%, 92%, 93%, 94%, 95%, 96%, 97%, 98%, 99% ou 100% avec ladite SEQ ID NO : 6, notamment 95% et plus particulièrement 98% ou 99%.

Le terme «gène *LSU* d'une algue» fait référence au gène codant la grande sous-unité d'un ribosome (*LSU* pour Large Sub-Unit). Il s'agit d'un gène d'origine nucléaire.

Le terme «détection» signifie que l'utilisation de la paire d'oligonucléotides susmentionnée permet de conclure quant à la présence ou à l'absence d'algue dans l'échantillon testé.

Le terme «identification» signifie que l'utilisation de la paire d'oligonucléotides susmentionnée permet d'associer l'algue présente dans l'échantillon testé à un embranchement et/ou à une classe et/ou à un ordre et/ou à une famille et/ou à un genre et/ou à une espèce d'algue. Dans un aspect préféré, le terme «identification» signifie que l'utilisation de la paire d'oligonucléotides susmentionnée permet d'associer l'algue présente dans l'échantillon testé à une espèce d'algue. Des exemples des embranchements, des classes, des ordres, des familles, des genres et des espèces d'algues sont communiqués dans le Tableau 1 ci-après.

L'expression «la détection et/ou l'identification» signifie «la détection» ou «l'identification» ou «la détection et l'identification», et plus particulièrement «l'identification».

L'expression «échantillon susceptible d'en contenir» signifie que l'échantillon peut comprendre ou peut ne pas comprendre d'algue, et notamment peut comprendre ou peut ne pas comprendre d'ADN d'algue.

Le terme «hybrider» signifie que les oligonucléotides formant la paire d'oligonucléotides (les amorces d'amplification) de la présente demande s'hybrident avec une séquence «marqueur» de référence du gène *LSU* (notamment les séquences de SEQ ID NO: 1, SEQ ID NO: 2, SEQ ID NO: 5 et SEQ ID NO: 6) à un niveau supérieur au bruit de fond de manière significative. Le niveau du signal généré par l'interaction entre la séquence capable de s'hybrider de manière sélective (les séquences formant la paire d'oligonucléotides) et les séquences de référence (les séquences cibles) est généralement 10 fois, de préférence 100 fois plus intense que celui de l'interaction des autres séquences d'ADN générant le bruit de fond. Les conditions d'hybridation stringentes permettant une hybridation sélective sont bien connues de l'homme du métier.

L'expression «conditions de stringence suffisantes» signifie par exemple une température d'hybridation de 40-65°C, de préférence de 50-60°C, notamment 53-58°C, et plus particulièrement 53°C, 55°C, 58°C dans un tampon d'amplification d'une concentration saline comprise de 0,001 à 0,5M notamment comprenant du MgCh 2mM.

La présente demande décrit, l'utilisation d'une paire d'amorces d'amplification, dans laquelle ledit premier oligonucléotide ou ledit deuxième oligonucléotide de la première ou de la deuxième paire d'amorces d'amplification s'hybride à sa séquence cible respective choisie parmi les séquences du groupe constitué par: SEQ ID NO: 1, SEQ ID NO:2 , SEQ ID NO: 5, et SEQ ID NO: 6 du gène *LSU* d'une algue, à une température comprise de 40 à 65°C, et plus particulièrement de 50-60°C et une concentration saline comprise de 0, 001 à 0,5 M, et plus particulièrement de 0.05 à 0,1 M, et encore plus particulièrement de à 0,001 à 0,005M.

L'expression «amplification d'au moins une région variable du gène *LSU* d'une algue» fait référence à toute amplification enzymatique *in vitro* d'une séquence définie d'ADN du gène *LSU* d'une algue, par exemple par une réaction d'amplification en chaîne par polymérase (PCR).

L'expression « ladite région variable constituant un marqueur de détection et/ou d'identification d'une algue » signifie que la région variable du gène *LSU* qui est amplifiée constitue une région d'ADN d'algue suffisamment courte et suffisamment variable pour pouvoir détecter et/ou identifier une algue, notamment à partir d'un échantillon contenant de l'ADN d'algue dégradé, et plus particulièrement pour identifier l'ADN d'une algue.

Dans un autre aspect particulier, la présente demande décrit l'utilisation d'une paire d'oligonucléotides (amorces d'amplification) dans laquelle :
- le premier oligonucléotide de la première paire d'amorces d'amplification a une séquence de 15 à 30 nucléotides contigus, de préférence de 18 à 24 nucléotides contigus choisie dans la séquence de SEQ ID NO: 3 et le deuxième oligonucléotide a une séquence de 15 à 30 nucléotides contigus, de préférence de 18 à 24 nucléotides contigus choisie dans la séquence de SEQ ID NO: 4,
- le premier oligonucléotide de la deuxième paire d'amorces d'amplification a une séquence de 15 à 30 nucléotides contigus, de préférence de 18 à 24 nucléotides contigus choisie dans la séquence de SEQ ID NO: 7 et le deuxième oligonucléotide a une séquence de 15 à 30 nucléotides contigus, de préférence de 18 à 24 nucléotides contigus choisie dans la séquence de SEQ ID NO: 8,
pour la détection et/ou l'identification d'une algue dans un échantillon susceptible d'en contenir, par amplification d'au moins une région variable du gène *LSU* d'une algue, ladite région variable constituant un marqueur de détection et/ou d'identification d'une algue, et dans laquelle ladite algue appartient à l'un des embranchements suivants : *Glaucophyta, Cyanobacteria, Dinophyta, Ochrophyta, Euglenozoa Charophyta, Chlorophyta* , ou *Rhodophyta.*

Le terme « *Glaucophyta* » fait référence à l'embranchement des organismes caractérisés par des chloroplastes, ou « cyanelles », de couleur bleu-vert, et par la présence d'alvéoles sous la membrane plasmique sous-tendues par des microtubules. Cet embranchement comporte par exemple la classe des *Glaucophyceae.*

Le terme « *Cyanobacteria* » fait référence à l'embranchement des bactéries également appelées « algues bleues », ou autrefois « algues bleu-vert ». Cet embranchement comporte l'unique classe des *Cyanophyceae.*

Le terme « *Dinophyta* » fait référence à l'embranchement des algues qui sont notamment caractérisées par un noyau particulier : le dinocaryon. Cet embranchement comporte par exemple la classe des *Dinophyceae.*

Le terme « *Ochrophyta* » fait référence à l'embranchement des algues brunes-dorées. Cet embranchement comporte par exemple la classe des *Phaeophyceae.*

Le terme « *Euglenozoa* » fait référence à l'embranchement des algues caractérisées par des crêtes mitochondriales discoïdes, et dont la membrane plasmique est sous-tendue par des feuillets de microtubules corticaux. Cet embranchement comporte par exemple la classe des *Euglenophyceae.*

Le terme « *Charophyta* » fait référence à une division des *Plantae.* Elles comprennent une partie des algues dites « algues vertes ». Cet embranchement comporte par exemple la classe des *Charophyceae.*

Le terme « *Chlorophyta* » fait référence à une division des *Plantae.* Elles comprennent une partie des algues dites « algues vertes ». Cet embranchement comporte par exemple la classe des *Ulvophyceae.*

Le terme « *Rodophyta* », dites « algues rouges » fait référence à l'embranchement des algues caractérisées par une composition pigmentaire avec un seul type de chlorophylle, la chlorophylle *a*, des caroténoïdes et des pigments caractéristiques, les phycobiliprotéines. Cet embranchement comporte par exemple la classe des *Cyanidiophyceae.*

Dans un autre aspect préféré, ledit premier oligonucléotide (amorce d'amplification) s'hybride à une séquence d'au moins 15 nucléotides contigus choisie dans la séquence de SEQ ID NO: 1 du gène *LSU* d'une algue et a une séquence d'au moins 15 nucléotides contigus choisie dans la séquence de SEQ ID NO: 3.

Dans un autre aspect préféré, ledit deuxième oligonucléotide (amorce d'amplification) s'hybride à une séquence d'au moins 15 nucléotides contigus choisie dans la séquence de SEQ ID NO: 2 du gène *LSU* d'une algue et a une séquence d'au moins 15 nucléotides contigus choisie dans la séquence de SEQ ID NO: 4.

L'expression «15 nucléotides contigus » signifie par exemple 15, 16, 17, 18, 19, 20, 21, 22, 23, 24, 25, 26, 27, 28, 29 ou 30 nucléotides contigus.

Le terme « SEQ ID NO : 1 » doit s'entendre comme une séquence de SEQ ID NO : 1 ou une séquence ayant un pourcentage d'identité d'au moins 80%, 81%, 82 %, 83%, 84%, 85%, 86%, 87%, 88%, 89%, 90%, 91%, 92%, 93%, 94%, 95%, 96%, 97%, 98%, 99% ou 100% avec ladite SEQ ID NO : 1, notamment 95% et plus particulièrement 98% ou 99%.

Le terme « SEQ ID NO : 2 » doit s'entendre comme une séquence de SEQ ID NO : 2 ou une séquence ayant un pourcentage d'identité d'au moins 80%, 81%, 82 %, 83%, 84%, 85%, 86%, 87%, 88%, 89%, 90%, 91%, 92%, 93%, 94%, 95%, 96%, 97%, 98%, 99% ou 100% avec ladite SEQ ID NO : 2, notamment 95% et plus particulièrement 98% ou 99%.

Dans un aspect particulier, la présente demande décrit l'utilisation d'une paire d'oligonucléotides dans laquelle le premier oligonucléotide (amorce d'amplification) ayant une séquence de 15 à 30 nucléotides contigus, de préférence de 18 à 24 nucléotides contigus choisie dans une séquence du groupe constitué par: SEQ ID NO: 3 est représenté par l'une des séquences suivantes: SEQ ID NO : 42, SEQ ID NO: 43, SEQ ID NO: 44, SEQ ID NO: 45, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO : 53, SEQ ID NO: 54, SEQ ID NO: 55, SEQ ID NO: 56 ou SEQ ID NO: 57. Bien entendu, lesdites séquences constituent des exemples non limitatifs de séquences de 18 à 24 nucléotides choisies dans la séquence de SEQ ID NO: 3 pouvant être utilisées dans le cadre de la présente demande.

Dans un aspect particulier, la présente demande décrit l'utilisation d'une paire d'oligonucléotides susmentionnée, dans laquelle le deuxième oligonucléotide ayant une séquence de 15 à 30 nucléotides contigus, de préférence de 18 à 24 nucléotides contigus choisie dans la séquence de SEQ ID NO : 4 est représenté par l'une des séquences suivantes : SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70. Bien entendu, lesdites séquences constituent des exemples non limitatifs de séquences de 18 à 24 nucléotides choisies dans la séquence de SEQ ID NO : 4 pouvant être utilisées dans le cadre de la présente demande.

Dans un aspect particulier, la présente demande décrit l'utilisation d'une paire d'oligonucléotides (amorces d'amplification) dans laquelle:
- le premier oligonucléotide qui s'hybride à une séquence de 15 à 30 nucléotides contigus, de préférence de 18 à 24 nucléotides contigus choisie dans la séquence de SEQ ID NO : 5 du gène *LSU* d'une algue a une séquence complémentaire de 18 à 24 nucléotides contigus choisie dans la séquence de SEQ ID NO : 7 et le deuxième oligonucléotide qui s'hybride à une séquence de 15 à 30 nucléotides contigus, de préférence de 18 à 24 nucléotides contigus choisie dans la séquence de SEQ ID NO : 6 du gène *LSU* d'une algue a une séquence complémentaire de 18 à 24 nucléotides contigus choisie dans la séquence de SEQ ID NO : 8,
pour la détection et/ou l'identification d'une algue dans un échantillon susceptible d'en contenir, par amplification d'au moins une région variable du gène *LSU* d'une algue, ladite région variable constituant un marqueur de détection et/ou d'identification d'une algue, et dans laquelle ladite algue appartient à l'un des embranchements suivants : *Glaucophyta, Dinophyta, Charophyta, Chlorophyta, Euglenozoa, Ochrophyta* ou *Rhodophyta.*

Le terme « SEQ ID NO : 5 » doit s'entendre comme une séquence de SEQ ID NO : 5 ou une séquence ayant un pourcentage d'identité d'au moins 80%, 81%, 82 %, 83%, 84%, 85%, 86%, 87%, 88%, 89%, 90%, 91%, 92%, 93%, 94%, 95%, 96%, 97%, 98%, 99% ou 100% avec ladite SEQ ID NO : 5, notamment 95% et plus particulièrement 98% ou 99%.

Le terme « SEQ ID NO : 6 » doit s'entendre comme une séquence de SEQ ID NO : 6 ou une séquence ayant un pourcentage d'identité d'au moins 80%, 81%, 82 %, 83%, 84%, 85%, 86%, 87%, 88%, 89%, 90%, 91%, 92%, 93%, 94%, 95%, 96%, 97%, 98%, 99% ou 100% avec ladite SEQ ID NO : 6, notamment 95% et plus particulièrement 98% ou 99%.

Dans un aspect particulier, la présente demande décrit l'utilisation d'une paire d'oligonucléotides dans laquelle le premier oligonucléotide ayant une séquence de 15 à 30 nucléotides contigus, de préférence de 18 à 24 nucléotides contigus choisie dans la séquence de SEQ ID NO : 7 est représenté par l'une des séquences suivantes : SEQ ID NO : 20, SEQ ID NO : 21, SEQ ID NO : 22, SEQ ID NO : 23, SEQ ID NO : 24, SEQ ID NO : 25, SEQ ID NO : 26, SEQ ID NO : 27, SEQ ID NO : 28 ou SEQ ID NO : 29.
Bien entendu, lesdites séquences constituent des exemples non limitatifs de séquence de 15 à 30 nucléotides contigus, de préférence de 18 à 24 nucléotides choisies dans la séquence de SEQ ID NO : 7 pouvant être utilisées dans le cadre de la présente demande.

Dans un aspect particulier, la présente demande décrit l'utilisation d'une paire d'oligonucléotides dans laquelle le deuxième oligonucléotide ayant une séquence de 15 à 30 nucléotides contigus, de préférence de 18 à 24 nucléotides contigus choisie dans la séquence de SEQ ID NO: 8 est représenté par l'une des séquences suivantes: SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 41.
Bien entendu, lesdites séquences constituent des exemples non limitatifs de séquence de 15 à 30 nucléotides contigus, de préférence de 18 à 24 nucléotides choisies dans la séquence de SEQ ID NO: 8 pouvant être utilisées dans le cadre de la présente demande.

Dans un aspect particulier, la présente demande décrit l'utilisation d'une paire d'oligonucléotides susmentionnée, dans laquelle ladite région variable du gène *LSU* d'une algue qui est amplifiée a une taille inférieure ou égale à 220 paires de bases, notamment inférieure ou égale à 200, 190, 180 et notamment 178 paires de bases.

L'expression « une taille inférieure ou égale à 200 paires de bases » signifie une taille d'environ 100 à 200 paires de bases, notamment 110, 115, 120, 125, 130, 135, 140, 145, 150, 155, 160, 165, 170, 175, 180, 185, 190, 195 ou 200 paires de bases.

Dans un aspect encore plus particulier, la présente demande décrit l'utilisation d'une paire d'oligonucléotides (amorces d'amplification) susmentionnée, dans laquelle ladite région variable du gène *LSU* d'une algue qui est amplifiée a une taille de 126 à 178 paires de bases.

L'expression « 126 à 178 paires de bases » signifie 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177 ou 178 paires de bases.

Dans un aspect particulier, la présente demande décrit l'utilisation d'une paire d'oligonucléotides susmentionnée, dans laquelle ladite région variable du gène *LSU* d'une algue est représentée par une séquence choisie dans le groupe constitué par: SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, ou une séquence ayant un pourcentage d'identité d'au moins 80% avec ladite séquence et notamment 95% et plus particulièrement 98% ou 99%.

Dans un aspect encore plus particulier, la présente demande décrit l'utilisation d'une paire d'amorces d'amplification, dans laquelle ladite région variable du gène LSU d'une algue est représentée par une séquence choisie dans le groupe constitué par:, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, ou une séquence ayant un pourcentage d'identité d'au moins 80% avec ladite séquence et notamment 95% et plus particulièrement 98% ou 99%.

Dans un aspect particulier, la présente demande décrit l'utilisation d'une paire d'oligonucléotides (amorces d'amplification) susmentionnée, dans laquelle ledit échantillon est un produit qui est susceptible de contenir au moins une molécule d'ADN d'algue.

Dans un aspect particulier, la présente demande décrit l'utilisation d'une paire d'oligonucléotides (amorces d'amplification) susmentionnée, dans laquelle ledit échantillon est un produit qui comprend ou consiste en au moins une molécule d'ADN d'algue.

Dans un aspect particulier, la présente demande décrit l'utilisation d'une paire d'oligonucléotides (amorces d'amplification) susmentionnée, dans laquelle ledit échantillon est un produit qui ne comprend pas au moins une molécule d'ADN d'algue.

Dans un aspect particulier, la présente demande décrit l'utilisation d'une paire d'oligonucléotides (amorces d'amplification) susmentionnée, dans laquelle ladite molécule d'ADN d'algue a une taille inférieure ou égale à 220 paires de bases.

L'expression « inférieure ou égale à 220 paires de bases » signifie par exemple de 100 à 220 paires de bases, notamment 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219 ou 220.

Dans un aspect encore plus particulier, lorsque ladite molécule d'ADN d'algue correspond au gène *LSU* d'une algue (ou un fragment dudit gène *LSU*)*,* alors la molécule d'ADN possède au maximum 220 paires de bases, selon la détermination ci-après: la séquence de l'amorce sens (20 paires de bases par exemple) + les fragments correspondant à la région amplifiée (126 à 178 paires de bases) + la séquence de l'amorce anti-sens (18 paires de bases par exemple). Bien entendu la détermination ci-dessus peut être modifiée, pour s'adapter par exemple à l'utilisation d'amorces faisant 21 paires de bases ; dans tous les cas ladite molécule d'ADN possède au maximum 220 paires de bases.

Dans un aspect particulier, la présente demande décrit l'utilisation d'une paire d'oligonucléotides susmentionnée, dans laquelle ledit échantillon est choisi parmi : un produit à base d'algues, un produit qui contient au moins une algue processée, un extrait d'algue, un mélange d'algues et de plantes, un mélange d'algues, un produit à base d'algues et de plantes, un produit qui ne contient pas d'algue, un mélange qui ne contient pas d'algue, un échantillon de plante sans algue ou une algue pure.

L'expression «produit à base d'algues» signifie un produit contenant au moins une algue dont l'intégrité de l'ADN n'a pas été altérée.

L'expression « un produit qui contient au moins une algue processée» signifie un produit contenant au moins une algue dont l'intégrité de l'ADN a été altérée.

L'expression « un extrait d'algues » signifie un échantillon obtenu par extraction d'une algue, c'est-à-dire un échantillon/extrait comprenant au moins de l'ADN d'une algue, l'intégrité dudit ADN étant altérée ou non altérée.

L'expression « un mélange d'algues et de plantes » signifie un mélange contenant au moins de l'ADN d'une algue et au moins de l'ADN d'une plante.

L'expression « un mélange d'algues » signifie un mélange contenant de l'ADN d'algue d'au moins deux algues appartenant à des embranchements différents et/ou des classes différentes et/ou des ordres différents et/ou des familles différentes et/ou des genres différents et/ou des espèces d'algues différentes, notamment des algues dont l'ADN a été processé.

L'expression « un produit à base d'algues et de plantes » signifie un produit contenant au moins de l'ADN d'une algue (processé ou non processé) et au moins de l'ADN d'une plante.

L'expression « un produit qui ne contient pas d'algue » signifie un produit contenant 0% d'ADN d'algue.

L'expression « un échantillon de plante sans algue » signifie un échantillon/extrait comprenant au moins de l'ADN d'une plante, mais ne contenant pas d'ADN d'algue (en d'autres termes ledit échantillon de plante contient 0% d'ADN d'algue).

L'expression « algue pure » signifie un produit contenant 100% d'ADN algue d'une seule espèce.

Dans un aspect particulier, la présente demande décrit l'utilisation d'une paire d'oligonucléotides (amorces d'amplification) susmentionnée, dans laquelle l'embranchement auquel appartient l'algue de ladite molécule d'ADN d'algue susceptible d'être présente dans le susdit échantillon est connu ou inconnu.

Dans un aspect particulier, la présente demande décrit l'utilisation d'une paire d'oligonucléotides (amorces d'amplification) susmentionnée, dans laquelle la classe à laquelle appartient l'algue de ladite molécule d'ADN d'algue susceptible d'être présente dans le susdit échantillon est connue ou inconnue.

Dans un aspect particulier, la présente demande décrit l'utilisation d'une paire d'oligonucléotides (amorces d'amplification) susmentionnée, dans laquelle l'ordre auquel appartient l'algue de ladite molécule d'ADN d'algue susceptible d'être présente dans le susdit échantillon est connue ou inconnue.

Dans un aspect particulier, la présente demande décrit l'utilisation d'une paire d'oligonucléotides (amorces d'amplification) susmentionnée, dans laquelle la famille à laquelle appartient l'algue de ladite molécule d'ADN d'algue susceptible d'être présente dans le susdit échantillon est connue ou inconnue.

Dans un aspect particulier, la présente demande décrit l'utilisation d'une paire d'oligonucléotides (amorces d'amplification) susmentionnée, dans laquelle le genre auquel appartient l'algue de ladite molécule d'ADN d'algue susceptible d'être présente dans le susdit échantillon est connue ou inconnue.

Dans un aspect particulier, la présente demande décrit l'utilisation d'une paire d'oligonucléotides (amorces d'amplification) susmentionnée, dans laquelle l'espèce à laquelle appartient l'algue de ladite molécule d'ADN d'algue susceptible d'être présente dans le susdit échantillon est connue ou inconnue.

Dans un aspect particulier, la présente demande décrit l'utilisation d'une paire d'oligonucléotides (amorces d'amplification) susmentionnée, dans laquelle ladite détection et/ou identification d'une algue est effectuée par hybridation dans des conditions de stringence suffisantes pour l'amplification de ladite région variable du gène *LSU* d'une algue.

L'expression « conditions de stringence suffisantes » signifie par exemple une température d'hybridation de 50-60°C, notamment 53-58°C, et plus particulièrement 53°C, 55°C, 58°C dans un tampon d'amplification d'une concentration saline comprise de 0,001 à 0,5M notamment comprenant du MgCh 2mM.

Dans un aspect particulier, la présente demande décrit l'utilisation d'une paire d'oligonucléotides (amorces d'amplification) susmentionnée, dans laquelle ladite hybridation dans des conditions de stringence suffisantes comprend ou consiste en une hybridation des susdits premier et deuxième oligonucléotides avec:
- des séquences de 15 à 30 nucléotides contigus, de préférence de 18 à 24 nucléotides contigus choisies dans les séquences de SEQ ID NO: 1, SEQ ID NO: 2, SEQ ID NO: 5 et SEQ ID NO: 6 du gène *LSU* d'une algue, à une température de 50-60°C, notamment 53-58°C et plus particulièrement 53°C, 55°C ou 58°C dans un tampon d'amplification d'une concentration saline comprise de 0,001 à 0,5M notamment comprenant du MgCh 2mM.

Dans un aspect particulier, la présente demande décrit l'utilisation d'une paire d'oligonucléotides (amorces d'amplification) susmentionnée, dans laquelle ladite hybridation dans des conditions de stringence suffisantes comprend ou consistent en une hybridation des susdits premier et deuxième oligonucléotides avec:
- des séquences de 15 à 30 nucléotides contigus, de préférence de 18 à 24 nucléotides contigus choisies dans les séquences de SEQ ID NO: 3, SEQ ID NO: 4, SEQ ID NO: 7 et SEQ ID NO: 8 du gène *LSU* d'une algue, à une température de 50-60°C, notamment 55°C dans un tampon d'amplification d'une concentration saline comprise de 0,001 à 0,5M notamment comprenant du MgCh 2mM.

Dans un aspect particulier, la présente demande décrit l'utilisation d'une paire d'oligonucléotides (amorces d'amplification) susmentionnée, dans laquelle ladite amplification d'au moins une région variable du gène *LSU* d'une algue comprend ou consiste en la répétition du cycle comprenant ou constitué de :
- une étape d'hybridation d'au moins une molécule d'ADN simple brin d'algue, issue de la dénaturation d'une molécule d'ADN double brin d'algue provenant d'un échantillon susceptible de contenir au moins une algue, avec au moins une amorce constituée par un des deux oligonucléotides formant la susdite paire d'oligonucléotides.

Dans un aspect particulier, la présente demande décrit l'utilisation d'une paire d'oligonucléotides susmentionnée, dans laquelle ladite amplification d'au moins une région variable du gène *LSU* d'une algue comprend ou consiste en la répétition du cycle comprenant ou constitué de :
- une étape d'hybridation d'au moins une molécule d'ADN simple brin d'algue, issue de la dénaturation d'une molécule d'ADN double brin d'algue provenant d'un échantillon susceptible de contenir au moins une algue, avec au moins une amorce constituée par un des deux oligonucléotides formant la susdite paire d'oligonucléotides,
- une étape d'élongation du brin complémentaire d'au moins une des deux susdites molécules d'ADN simple brin d'algues, par l'ADN polymérase, à partir de la susdite amorce, afin d'obtenir un produit d'amplification constituant un marqueur de détection et/ou d'identification d'une algue.

Le produit d'amplification correspond ici à la région variable amplifiée du gène *LSU* d'une algue qui constitue un marqueur dit « court » au sens de la présente demande.

Dans un aspect particulier de la présente demande, l'utilisation d'une paire d'oligonucléotides susmentionnée, comprenant ou consistant en la répétition dudit cycle comprenant ou étant constitué d'une étape d'hybridation et d'une étape d'élongation, doit également s'entendre comme un procédé d'amplification.

Dans un aspect particulier, la présente demande décrit l'utilisation d'une paire d'oligonucléotides susmentionnée, dans laquelle ladite amplification d'au moins une région variable du gène *LSU* d'une algue comprend ou consiste en la répétition du cycle comprenant ou constitué de:
- une étape de dénaturation d'une molécule d'ADN double brin d'algue provenant d'un échantillon susceptible de contenir au moins une algue afin d'obtenir deux molécules d'ADN simple brin d'algue,
- une étape d'hybridation d'au moins une des deux susdites molécules d'ADN simple brin d'algue avec au moins une amorce constituée par un des deux oligonucléotides formant la susdite paire d'oligonucléotides,
- une étape d'élongation du brin complémentaire d'au moins une des deux susdites molécules d'ADN simple brin d'algue, par l'ADN polymérase, à partir de la susdite amorce, afin d'obtenir un produit d'amplification constituant un marqueur de détection et/ou d'identification d'une algue.

Dans un aspect particulier, la présente demande décrit l'utilisation d'une paire d'oligonucléotides (amorces d'amplification) susmentionnée, dans laquelle ladite amplification d'au moins une région variable du gène *LSU* d'une algue comprend ou consiste en la répétition du cycle comprenant ou constitué de:
- une étape d'hybridation de deux molécules d'ADN simple brin d'algue, issues de la dénaturation d'une molécule d'ADN double brin d'algue provenant d'un échantillon susceptible de contenir au moins une algue, avec deux amorces constituées par les deux oligonucléotides formant la susdite paire d'oligonucléotides.

Dans un aspect particulier, la présente demande décrit l'utilisation d'une paire d'oligonucléotides (amorces d'amplification) susmentionnée, dans laquelle ladite amplification d'au moins une région variable du gène *LSU* d'une algue comprend ou consiste en la répétition du cycle comprenant ou constitué de:
- une étape d'hybridation de deux molécules d'ADN simple brin d'algue, issues de la dénaturation d'une molécule d'ADN double brin d'algue provenant d'un échantillon susceptible de contenir au moins une algue, avec deux amorces constituées par les deux oligonucléotides formant la susdite paire d'oligonucléotides,
- une étape d'élongation des brins complémentaires des deux susdites molécules d'ADN simple brin d'algues, par l'ADN polymérase, à partir des susdites amorces, afin d'obtenir un produit d'amplification constituant un marqueur de détection et/ou d'identification d'une algue.

Dans un aspect particulier, la présente demande décrit l'utilisation d'une paire d'oligonucléotides (amorces d'amplification) susmentionnée, dans laquelle ladite amplification d'au moins une région variable du gène *LSU* d'une algue comprend ou consiste en la répétition du cycle comprenant ou constitué de:
- une étape de dénaturation d'une molécule d'ADN double brin d'algue provenant d'un échantillon susceptible de contenir au moins une algue afin d'obtenir deux molécules d'ADN simple brin d'algue,
- une étape d'hybridation de deux susdites molécules d'ADN simple brin d'algues avec deux amorces constituées par les deux oligonucléotides formant la susdite paire d'oligonucléotides,
- une étape d'élongation des brins complémentaires des deux susdites molécules d'ADN simple brin d'algues, par l'ADN polymérase, à partir des susdites amorces, afin d'obtenir un produit d'amplification constituant un marqueur de détection et/ou d'identification d'une algue.

Dans un aspect particulier, la présente demande décrit l'utilisation d'une paire d'oligonucléotides (amorces d'amplification) susmentionnée, dans laquelle ladite répétition du cycle est une répétition d'au moins 25 cycles, notamment 40 cycles et plus particulièrement 49, 50 ou 51 cycles.

L'expression « au moins 25 cycles » signifie par exemple 25, 26, 27, 28, 29, 30, 31, 32, 33, 34, 35, 36, 37, 38, 39, 40, 41, 42, 43, 44, 45, 46, 47, 48, 49, 50 ou 51 cycles.

Dans un aspect particulier, la présente demande décrit l'utilisation d'une paire d'oligonucléotides (amorces d'amplification) susmentionnée, pour la détection et/ou l'identification d'une algue appartenant à l'embranchement des *Chlorophyta,* à l'exception des algues de l'ordre des *Bryopsidales.*

La présente demande décrit un oligonucléotide (amorce d'amplification) ayant une taille comprise de 15 à 45 nucléotides, notamment de 15 à 30 nucléotides contigus, de préférence de 18 à 24 nucléotides contigus, ledit oligonucléotide comprenant ou étant constitué par une séquence complémentaire d'une séquence choisie parmi:
- SEQ ID NO: 1, ou une séquence ayant un pourcentage d'identité d'au moins 80%, 81%, 82%, 83%, 84%, 85%, 86%, 86%, 88%, 89%, 90%, 91%, 92%, 93%, 94%, 95%, 96%, 97%, 98%, 99% avec ladite séquence de SEQ ID NO: 1, ou
- SEQ ID NO: 2, ou une séquence ayant un pourcentage d'identité d'au moins 80%, 81%, 82%, 83%, 84%, 85%, 86%, 86%, 88%, 89%, 90%, 91%, 92%, 93%, 94%, 95%, 96%, 97%, 98%, 99% avec ladite séquence de SEQ ID NO: 2, ou
- SEQ ID NO: 5, ou une séquence ayant un pourcentage d'identité d'au moins 80%, 81%, 82%, 83%, 84%, 85%, 86%, 86%, 88%, 89%, 90%, 91%, 92%, 93%, 94%, 95%, 96%, 97%, 98%, 99% avec ladite séquence de SEQ ID NO: 5, ou
- SEQ ID NO: 6, ou une séquence ayant un pourcentage d'identité d'au moins 80%, 81%, 82%, 83%, 84%, 85%, 86%, 86%, 88%, 89%, 90%, 91%, 92%, 93%, 94%, 95%, 96%, 97%, 98%, 99% avec ladite séquence de SEQ ID NO: 6,
permettant d'amplifier un fragment d'une taille inférieure ou égale à 220 paires de bases d'une région variable du gène LSU codant pour la grande sous-unité d'un ribosome, d'une algue.

Ces oligonucléotides correspondent aux séquences cibles des amorces de la présente demande.

Dans un aspect encore plus particulier, la présente demande décrit également un oligonucléotide (amorce d'amplification) ayant une taille comprise de 15 à 45 nucléotides, notamment de 15 à 30 nucléotides contigus, de préférence de 18 à 24 nucléotides contigus, ledit oligonucléotide comprenant ou étant constitué par une séquence choisie parmi :
- SEQ ID NO : 1, ou une séquence ayant un pourcentage d'identité d'au moins 80% avec ladite séquence de SEQ ID NO : 1, notamment 95% et plus particulièrement 98% ou 99%, ou
- SEQ ID NO : 2, ou une séquence ayant un pourcentage d'identité d'au moins 80% avec ladite séquence de SEQ ID NO : 2, notamment 95% et plus particulièrement 98% ou 99%.

Dans un aspect encore plus particulier, la présente demande décrit également un oligonucléotide (amorce d'amplification) ayant une taille comprise de 15 à 45 nucléotides, notamment de 15 à 30 nucléotides contigus, de préférence de 18 à 24 nucléotides contigus, ledit oligonucléotide comprenant ou étant constitué par une séquence choisie parmi :
- SEQ ID NO : 5, ou une séquence ayant un pourcentage d'identité d'au moins 80% avec ladite séquence de SEQ ID NO : 5, notamment 95% et plus particulièrement 98% ou 99%, ou
- SEQ ID NO : 6, ou une séquence ayant un pourcentage d'identité d'au moins 80% avec ladite séquence de SEQ ID NO : 6, notamment 95% et plus particulièrement 98% ou 99%.

L'expression « une taille de 15 à 45 nucléotides » signifie une taille de 15, 16, 17, 18, 19, 20, 21, 22, 23, 24, 25, 26, 27, 28, 29, 30, 31, 32, 33, 34, 35, 36, 37, 38, 39, 40, 41, 42, 43, 44 ou 45 nucléotides.

La présente demande décrit un oligonucléotide (amorce d'amplification) ayant une taille comprise de 15 à 30, de préférence de 18 à 24 nucléotides contigus, choisi dans la séquence de SEQ ID NO: 7 ou SEQ ID NO: 8 comprenant ou étant constituée par une séquence choisie parmi SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 41, ou choisi dans la séquence de SEQ ID NO: 3 ou SEQ ID NO: 4 comprenant ou étant constituée par une séquence choisie parmi SEQ ID NO: 42, SEQ ID NO: 43, SEQ ID NO: 44, SEQ ID NO: 45, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, ou une séquence ayant un pourcentage d'identité d'au moins 81%, 82%, 83%, 84%, 85%, 86%, 86%, 88%, 89%, 90%, 91%, 92%, 93%, 94%, 95%, 96%, 97%, 98%, 99% ou 100% avec lesdites séquences.

La présente demande décrit une paire d'oligonucléotides ayant une taille comprise de 15 à 30, de préférence de 18 à 24 nucléotides contigus, lesdites paires étant constituées des couples d'oligonucléotides choisis parmi les couples de séquences:
- SEQ ID NO: 3 et une séquence de 18 à 24 nucléotides contigus choisie dans la séquence de SEQ ID NO: 4, et notamment choisie parmi SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70 ou
- SEQ ID NO: 4 et une séquence de 18 à 24 nucléotides contigus choisie dans la séquence de SEQ ID NO: 3, et notamment choisie parmi SEQ ID NO: 42, SEQ ID NO: 43, SEQ ID NO : 44, SEQ ID NO: 45, SEQ ID NO : 46, SEQ ID NO : 47, SEQ ID NO : 48, SEQ ID NO : 49, SEQ ID NO : 50, SEQ ID NO : 51, SEQ ID NO: 52, SEQ ID NO : 53, SEQ ID NO : 54, SEQ ID NO : 55, SEQ ID NO : 56, SEQ ID NO : 57, ou
- SEQ ID NO: 7 et une séquence de 18 à 24 nucléotides contigus choisie dans la séquence de SEQ ID NO: 8, et notamment choisie parmi SEQ ID NO: 30, SEQ ID NO: 31, SEQ ID NO: 32, SEQ ID NO: 33, SEQ ID NO: 34, SEQ ID NO: 35, SEQ ID NO : 36, SEQ ID NO : 37, SEQ ID NO: 38, SEQ ID NO: 39, SEQ ID NO: 40, SEQ ID NO : 41, ou
- SEQ ID NO: 8 et une séquence de 18 à 24 nucléotides contigus choisie dans la séquence de SEQ ID NO: 7, et notamment choisie parmi SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29.

Ces oligonucléotides correspondent aux séquences des couples des amorces de la présente demande.

Dans un aspect encore plus particulier, la présente demande décrit les couples d'amorces suivants:
- une séquence de 18 à 24 nucléotides contigus choisie dans la séquence SEQ ID NO: 3, et notamment choisie parmi SEQ ID NO: 42, SEQ ID NO: 43, SEQ ID NO: 44, SEQ ID NO: 45, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, et une séquence de 18 à 24 nucléotides contigus choisie dans la séquence SEQ ID NO: 4, et notamment choisie parmi SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70,
- et notamment la paire SEQ ID NO: 42 et SEQ ID NO: 58.

Dans un aspect encore plus particulier, la présente demande décrit les couples d'amorces suivants:
- une séquence de 18 à 24 nucléotides contigus choisie dans la séquence SEQ ID NO: 7 et notamment choisie parmi SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO : 37, SEQ ID NO: 38, SEQ ID NO: 39, SEQ ID NO: 40, SEQ ID NO : 41, ou
- SEQ ID NO: 8 et une séquence de 18 à 24 nucléotides contigus choisie dans la séquence de SEQ ID NO: 7, et notamment choisie parmi SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29,
- et notamment la paire SEQ ID NO: 20 et SEQ ID NO: 30.

Ces oligonucléotides correspondent aux séquences des couples des amorces LSU de la présente demande.

La présente demande décrit un polynucléotide (un marqueur de détection et/ou d'identification d'une algue) ayant une taille inférieure ou égale à 220 nucléotides d'une région variable amplifiée du gène LSU codant pour la grande sous-unité d'un ribosome, d'une algue, notamment inférieure ou égale à 190, 180 et notamment 178 nucléotides, ledit marqueur comprenant ou étant constitué par une séquence ayant un pourcentage d'identité d'au moins 80%, 81%, 82%, 83%, 84%, 85%, 86%, 86%, 88%, 89%, 90%, 91%, 92%, 93%, 94%, 95%, 96%, 97%, 98%, 99%, ou 100% avec une séquence choisie parmi SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, et SEQ ID NO: 19.

La présente demande décrit un polynucléotide (un marqueur de détection et/ou d'identification d'une algue) ayant une taille inférieure ou égale à 220 nucléotides d'une région variable amplifiée du gène LSU codant pour la grande sous-unité d'un ribosome, d'une algue, notamment inférieure ou égale à 190, 180 et notamment 178 nucléotides, ledit marqueur comprenant ou étant constitué par une séquence ayant un pourcentage d'identité d'au moins 98%, 99%, ou 100% avec une séquence choisie parmi, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, et SEQ ID NO: 19.

Ces polynucléotides correspondent aux séquences des régions amplifiées selon la présente demande.

La présente demande décrit l'utilisation d'au moins une région variable du gène *LSU* d'une algue pour la détection et/ou l'identification d'une algue, dans laquelle ladite région variable constitue un marqueur de détection et/ou d'identification d'une algue et est située aux positions:
- 267 à 410 sur le gène *LSU* des algues chez *Fucus vesiculosus.*

Les positions données sur le gène *LSU* s'entendent par rapport aux séquences générées: la séquence SEQ ID NO: 9 pour le gène *LSU* chez *Fucus vesiculosus* (la position 1 correspond au premier nucléotide suivant l'amorce T16N).

Les positions d'intérêt sur le gène *LSU,* pour les espèces autres que *Fucus vesiculosus* sont communiquées dans le Tableau 3 ci-après.

Dans un aspect particulier, dans ladite utilisation d'au moins une région variable du gène *LSU* d'une algue pour la détection et/ou l'identification d'une algue, dans laquelle ladite région variable constitue un marqueur de détection et/ou d'identification d'une algue et est un polynucléotide ayant une taille inférieure ou égale à 220 nucléotides, notamment inférieure ou égale à 178 nucléotides, ladite région variable du gène *LSU* d'une algue comprend ou est constituée par une séquence choisie parmi SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, et SEQ ID NO: 19,

La présente demande décrit un procédé d'amplification d'au moins une région variable du gène LSU d'une algue, ladite région variable constituant un marqueur de détection et/ou d'identification d'une algue, comprenant ou étant constitué des étapes suivantes:
a) une étape d'extraction du matériel nucléique d'un produit ou échantillon susceptible de contenir une algue,
b) une étape d'amplification de ladite région variable du gène LSU d'une algue, en utilisant -une première paire d'amorces d'amplification consiste en un premier oligonucléotide qui s'hybride à une séquence de 15 à 30 nucléotides contigus, de préférence de 18 à 24 nucléotides contigus choisie dans la séquence de SEQ ID NO: 1 du gène LSU d'une algue et un deuxième oligonucléotide qui s'hybride à une séquence de 15 à 30 nucléotides contigus, de préférence de 18 à 24 nucléotides contigus choisie dans la séquence de SEQ ID NO: 2 du gène LSU d'une algue, ou
   - une deuxième paire d'amorces d'amplification consiste en un premier oligonucléotide qui s'hybride à une séquence de 15 à 30 nucléotides contigus, de préférence de 18 à 24 nucléotides contigus choisie dans la séquence de SEQ ID NO: 5 du gène LSU d'une algue et le deuxième oligonucléotide s'hybride à une séquence de 15 à 30 nucléotides contigus, de préférence de 18 à 24 nucléotides contigus choisie dans la séquence de SEQ ID NO: 6 du gène LSU d'une algue,
c) une étape de détermination ou d'identification de la présence d'une algue dans ledit produit ou échantillon par la détection d'au moins une région variable amplifiée du gène LSU d'une algue.

L'expression « afin d'obtenir éventuellement au moins une région variable amplifiée du gène *LSU* d'une algue » signifie que le procédé d'amplification peut permettre d'obtenir au moins une région variable amplifiée du gène *LSU* d'une algue, ou peut ne pas permettre d'obtenir au moins une région variable amplifiée du gène *LSU* d'une algue. Dans ce dernier cas, la non-obtention d'au moins une région variable amplifiée du gène *LSU* d'une algue signifie qu'il n'y a pas d'ADN d'algue dans l'échantillon testé.

Dans un aspect particulier, la présente demande décrit un procédé d'amplification d'au moins une région variable du gène *LSU* d'une algue, ladite région variable amplifiée du gène LSU codant pour la grande sous-unité d'un ribosome d'une taille inférieure ou égale à 220 paires de bases constituant un marqueur de détection et/ou d'identification d'une algue, comprenant ou étant constitué des étapes suivantes :
a) une étape d'hybridation, dans des conditions de stringence suffisantes pour l'amplification de ladite région variable du gène *LSU* d'une algue, de deux molécules d'ADN simple brin d'algue, issue de la dénaturation d'une molécule d'ADN double brin d'algue provenant d'un échantillon susceptible de contenir au moins une algue, avec deux amorces constituées par les paires d'oligonucléotides choisies parmi :
   - un premier oligonucléotide s'hybridant à une séquence de 15 à 30 nucléotides contigus, de préférence de 18 à 24 contigus choisie dans la séquence de SEQ ID NO: 1 du gène *LSU* d'une algue et un deuxième oligonucléotide s'hybridant à une séquence de 15 à 30 nucléotides contigus, de préférence de 18 à 24 nucléotides contigus choisie dans la séquence de SEQ ID NO: 2 du gène *LSU* d'une algue, ou
   - un premier oligonucléotide s'hybridant à une séquence de 15 à 30 nucléotides contigus, de préférence de 18 à 24 nucléotides contigus choisie dans la séquence de SEQ ID NO: 5 du gène *LSU* d'une algue et un deuxième oligonucléotide s'hybridant à une séquence de 15 à 30 nucléotides contigus, de préférence de 18 à 24 nucléotides contigus choisie dans la séquence de SEQ ID NO: 6 du gène *LSU* d'une algue
   afin d'obtenir éventuellement au moins une région variable amplifiée du *LSU* d'une algue.

Dans un aspect particulier, la présente demande décrit un procédé d'amplification susmentionné, dans lequel ledit procédé comprend après ladite étape d'hybridation une étape d'élongation du brin complémentaire d'au moins une des deux susdites molécules d'ADN simple brin d'algue, par l'ADN polymérase, à partir de la susdite amorce, afin d'obtenir au moins une région variable amplifiée du gène *LSU* d'une algue.

Dans un aspect particulier, la présente demande décrit un procédé d'amplification susmentionné, dans lequel ledit procédé comprend après ladite étape d'hybridation une étape d'élongation de chacun des brins complémentaires des deux susdites molécules d'ADN simple brin d'algue, par l'ADN polymérase, à partir des susdites amorces, afin d'obtenir deux régions variables amplifiées du gène *LSU* d'une algue.

Dans un aspect particulier, la présente demande décrit un procédé d'amplification susmentionné, dans lequel ledit procédé comprend avant ladite étape d'hybridation une étape de dénaturation d'une molécule d'ADN double brin d'algue provenant d'un échantillon susceptible de contenir au moins une algue, afin d'obtenir deux molécules d'ADN simple brin d'algue.

Dans un aspect particulier, la présente demande décrit un procédé d'amplification susmentionné, dans lequel ladite étape de dénaturation d'une molécule d'ADN double brin d'algue est effectuée à une température d'environ 95°C pendant environ 30 secondes.

Dans un aspect particulier, la présente demande décrit un procédé d'amplification susmentionné, dans lequel ladite étape d'hybridation est effectuée à une température d'environ 50-60°C, notamment d'environ 53-55°C pendant environ 30 secondes.

Le terme « 50-60°C » signifie 50, 51, 52, 53, 54, 55, 56, 57, 58, 59, 60°C.

L'expression « environ 50-60°C » signifie que des valeurs proches de 50°C ou 60°C, telles que 49°C ou 61°C sont également comprises dans la portée de l'demande.

L'expression « environ 30 secondes » signifie 30 secondes ou des valeurs proches de 30 secondes, telles que 29 secondes ou 31 secondes.

Dans un aspect particulier, la présente demande décrit un procédé d'amplification susmentionné, dans lequel ladite étape d'hybridation est effectuée à une température d'environ 50-60°C, notamment environ 55°C pendant environ 30 secondes. Dans cet aspect particulier, ce sont les amorces LSU qui sont utilisées.

Dans un aspect particulier, la présente demande décrit un procédé d'amplification susmentionné, dans lequel ladite étape d'élongation est effectuée à une température d'environ 72°C pendant environ 1 minute.

Bien entendu la durée d'hybridation, la durée d'élongation et les températures d'hybridation et d'élongation dépendent de la longueur du fragment à amplifier et de la vitesse de l'enzyme, et peuvent être adaptées en fonction de la polymérase utilisée. Dans le cas de la présente demande les durées et températures sont données en référence à la Taq polymérase utilisée (Hot start FIREPol ; Solis).

L'expression « environ 72°C» signifie 72°C ou des valeurs proches de 72°C, telles que 71°C ou 73°C.

L'expression «environ 1 minute» signifie 60 secondes ou des valeurs proches de 60 secondes, telles que 59 secondes ou 61 secondes.

Dans un aspect particulier, la présente demande décrit un procédé d'amplification susmentionné, dans lequel ladite étape de dénaturation est précédée d'une étape de dénaturation initiale d'une molécule d'ADN double brin d'algue afin d'obtenir deux molécules d'ADN simple brin d'algue, à une température d'environ 95°C pendant environ 10 minutes.

Cette étape de dénaturation a pour objectif d'activer l'enzyme et de dénaturer la molécule d'ADN. Cette étape peut être optionnelle, en ce sens que certaines enzymes peuvent se passer de cette étape de dénaturation initiale.

La durée et température de cette étape de dénaturation dépendent de la polymérase utilisée (Taq polymérase - Hot start FIREPol; Solis dans le cas de la présente demande).

L'expression « environ 95°C» signifie 95°C ou des valeurs proches de 95°C, telles que 94°C ou 96°C.

L'expression « environ 10 minutes » signifie 10 minutes (ou 600 secondes) ou des valeurs proches de 600 secondes, telles que 550 secondes, 559 secondes, 610 secondes ou 601 secondes.

Dans un aspect particulier, la présente demande décrit un procédé d'amplification susmentionné, dans lequel ladite étape d'élongation est suivie d'une étape d'élongation finale du brin complémentaire d'au moins une des susdites molécules d'ADN simple brin d'algue, à une température d'environ 72°C pendant environ 7 minutes.

L'expression « environ 7 minutes» signifie 7 minutes (ou 420 secondes) ou des valeurs proches de 420 secondes, telles que 410 secondes, 419 secondes, 430 secondes ou 429 secondes.

La durée et température de cette étape d'élongation finale dépendent de la polymérase utilisée (Taq polymérase - Hot start FIREPol ; Solis dans le cas de la présente demande).

Dans un aspect particulier, la présente demande décrit un procédé d'amplification susmentionné, dans lequel ladite étape d'élongation finale est suivie d'une étape de diminution de la température au cours de laquelle la température est diminuée à une température d'environ 10°C.

L'expression « environ 10°C » signifie 10°C ou des valeurs proches de 10°C, telles que 9°C ou 11°C.

Cette diminution de température a pour objectif de stopper la réaction enzymatique, ramener l'échantillon à une température convenable pour travailler (sans se brûler) et conserver l'échantillon si jamais nécessaire.

Dans un aspect particulier, la présente demande décrit un procédé d'amplification susmentionné, dans lequel ledit procédé d'amplification d'au moins une région variable du gène *LSU* comprend ou est constitué des étapes suivantes :
- ladite étape de dénaturation d'une molécule d'ADN double brin d'algue, à une température d'environ 95°C pendant environ 30 secondes, afin d'obtenir deux molécules d'ADN simple brin d'algue,
- ladite étape d'hybridation d'au moins une des deux susdites molécules d'ADN simple brin, à une température d'environ 50-60°C, notamment environ 53-55°C pendant environ 30 secondes,
- ladite étape d'élongation du brin complémentaire d'au moins une des deux susdites molécules d'ADN simple brin d'algue, à une température d'environ 72°C pendant environ 1 minute, afin d'obtenir au moins une région variable amplifiée du gène *LSU.*

Dans un aspect particulier, la présente demande décrit un procédé d'amplification susmentionné, dans lequel ledit procédé d'amplification d'au moins une région variable du gène *LSU* comprend ou est constitué des étapes suivantes :
- ladite étape de dénaturation initiale d'une molécule d'ADN double brin, à une température de 95°C pendant 10 minutes, suivie de ladite étape de dénaturation d'une molécule d'ADN double brin d'algue, à une température d'environ 95°C pendant environ 30 secondes, afin d'obtenir deux molécules d'ADN simple brin d'algue,
- ladite étape d'hybridation d'au moins une des deux susdites molécules d'ADN simple brin, à une température d'environ 50-60°C, notamment environ 53-55°C pendant environ 30 secondes,
- ladite étape d'élongation du brin complémentaire d'au moins une des deux susdites molécules d'ADN simple brin d'algue, à une température d'environ 72°C pendant environ 1 minute, suivie de ladite étape d'élongation finale du brin complémentaire d'au moins une des deux susdites molécules d'ADN simple brin d'algue, à une température d'environ 72°C pendant environ 7 minutes, afin d'obtenir au moins une région variable amplifiée du gène *LSU* d'une algue,
- ladite étape de diminution de la température à une température d'environ 10°C.

Dans un aspect particulier, la présente demande décrit un procédé d'amplification susmentionné, dans lequel ledit procédé d'amplification d'au moins une région variable du gène *LSU* comprend ou est constitué des étapes suivantes :
- ladite étape de dénaturation d'une molécule d'ADN double brin d'algue, à une température d'environ 95°C pendant environ 30 secondes, afin d'obtenir deux molécules d'ADN simple brin d'algue,
- ladite étape d'hybridation d'au moins une des deux susdites molécules d'ADN simple brin, à une température d'environ 50-60°C, notamment environ 55°C pendant environ 30 secondes,
- ladite étape d'élongation du brin complémentaire d'au moins une des deux susdites molécules d'ADN simple brin d'algue, à une température d'environ 72°C pendant environ 1 minute, afin d'obtenir au moins une région variable amplifiée du gène *LSU.*

Dans un aspect particulier, la présente demande décrit un procédé d'amplification susmentionné, dans lequel ledit procédé d'amplification d'au moins une région variable du gène *LSU* comprend ou est constitué des étapes suivantes :
- ladite étape de dénaturation initiale d'une molécule d'ADN double brin, à une température d'environ 95°C pendant environ 10 minutes, suivie de ladite étape de dénaturation d'une molécule d'ADN double brin d'algue, à une température d'environ 95°C pendant environ 30 secondes, afin d'obtenir deux molécules d'ADN simple brin d'algue,
- ladite étape d'hybridation d'au moins une des deux susdites molécules d'ADN simple brin, à une température d'environ 50-60°C, notamment environ 55°C pendant environ 30 secondes,
- ladite étape d'élongation du brin complémentaire d'au moins une des deux susdites molécules d'ADN simple brin d'algue, à une température d'environ 72°C pendant environ 1 minute, suivie de ladite étape d'élongation finale du brin complémentaire d'au moins une des deux susdites molécules d'ADN simple brin d'algue, à une température d'environ 72°C pendant environ 7 minutes, afin d'obtenir au moins une région variable amplifiée du gène *LSU* d'une algue,
- ladite étape de diminution de la température à une température d'environ 10°C.

Dans un aspect particulier, la présente demande décrit un procédé d'amplification susmentionné, dans lequel ledit procédé comprend l'obtention d'au moins une région variable amplifiée du gène *LSU* d'une algue.

Dans un aspect encore plus particulier, la présente demande décrit un procédé d'amplification susmentionné, dans lequel ledit procédé comprend l'obtention d'au moins une région variable amplifiée du gène *LSU* d'une algue.

Dans un aspect particulier, la présente demande décrit un procédé d'amplification susmentionné, dans lequel ledit procédé ne permet pas l'obtention d'au moins une région variable amplifiée du gène *LSU* d'une algue.

Dans un aspect encore plus particulier, la présente demande décrit un procédé d'amplification susmentionné, dans lequel ledit procédé ne permet pas l'obtention d'au moins une région variable amplifiée du gène *LSU* d'une algue.

Dans un aspect particulier, la présente demande décrit un procédé d'amplification susmentionné, dans lequel ledit procédé comprend une étape de détection de ladite obtention d'au moins une région variable amplifiée du gène *LSU* d'une algue afin d'obtenir une région variable amplifiée et détectée du gène *LSU* d'une algue.

Dans un aspect encore plus particulier, la présente demande décrit un procédé d'amplification susmentionné, dans lequel ledit procédé comprend une étape de détection de ladite obtention d'au moins une région variable amplifiée du gène *LSU* d'une algue afin d'obtenir une région variable amplifiée et détectée du gène *LSU* d'une algue.

Ladite étape de détection d'au moins une région variable amplifiée du gène *LSU* d'une algue peut se faire par diverses techniques, notamment par électrophorèse.

Dans un aspect particulier, la présente demande décrit un procédé d'amplification susmentionné, dans lequel ledit procédé comprend une étape d'identification d'une algue par séquençage de ladite région variable amplifiée et détectée du gène *LSU* d'une algue afin d'obtenir une région variable amplifiée séquencée.

Dans un aspect particulier, la présente demande décrit un procédé d'amplification susmentionné, dans lequel ledit séquençage est un séquençage capillaire ou un séquençage NGS (Next Generation-Sequencing).

Dans un aspect particulier, la présente demande décrit un procédé d'amplification susmentionné, dans lequel ledit séquençage permet d'identifier l'embranchement auquel appartient l'algue par comparaison entre ladite région variable amplifiée séquencée et une séquence de référence.

Dans un aspect particulier, la présente demande décrit un procédé d'amplification susmentionné, dans lequel ledit séquençage permet d'identifier la classe à laquelle appartient l'algue par comparaison entre ladite région variable amplifiée séquencée et une séquence de référence.

Dans un aspect particulier, la présente demande décrit un procédé d'amplification susmentionné, dans lequel ledit séquençage permet d'identifier l'ordre auquel appartient l'algue par comparaison entre ladite région variable amplifiée séquencée et une séquence de référence.

Dans un aspect particulier, la présente demande décrit un procédé d'amplification susmentionné, dans lequel ledit séquençage permet d'identifier la famille à laquelle appartient l'algue par comparaison entre ladite région variable amplifiée séquencée et une séquence de référence.

Dans un aspect particulier, la présente demande décrit un procédé d'amplification susmentionné, dans lequel ledit séquençage permet d'identifier le genre appartient l'algue par comparaison entre ladite région variable amplifiée séquencée et une séquence de référence.

Dans un aspect particulier, la présente demande décrit un procédé d'amplification susmentionné, dans lequel ledit séquençage permet d'identifier l'espèce à laquelle appartient l'algue par comparaison entre ladite région variable amplifiée séquencée et une séquence de référence.

Dans un aspect particulier, ladite séquence de référence est choisie parmi les séquences suivantes SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, et SEQ ID NO: 19.

Dans un aspect particulier, la présente demande décrit un procédé d'amplification susmentionné, dans lequel la nature de l'échantillon est connue ou non.

Dans un aspect particulier, la présente demande décrit un procédé d'amplification susmentionné, dans lequel l'échantillon est un mélange d'algues, un mélange d'algues et de plantes, un produit à base d'algues, un produit à base d'algues et de plantes, un produit qui contient au moins une algue processée, un extrait d'algue, un produit qui ne contient pas d'algue, un échantillon de plante sans algue, un mélange sans algue, une algue pure ou un fragment d'algue pure ayant une molécule d'ADN dont la taille est inférieure ou égale à 220 nucléotides, notamment 200 nucléotides.

Dans un aspect particulier, les marqueurs dits courts selon la présente demande sont notamment utilisés pour analyser des échantillons d'ADN contenant une molécule d'ADN dont la taille est inférieure ou égale à 220 nucléotides.

**Tableau 1 : Exemple de classification d'algues**

| Domaine | Regne | Embranchement | Classe | Ordre | Famille | Espèce |
|---|---|---|---|---|---|---|
| Eubacteria | Bacteria | Cyanobacteria (Cyanobactéries) | Cyaniphyceae | Oscillatoriales | Phormidiaceae | Arthrospira sp. |
| Eukaryota | Chromista | Dinophyta (Dinoflagellées) | Dinophyceae | Suessiales | Suessiaceae | Symiodinium microadriaticum |
| | | Ochrophyta (Algues brunes) | Phaeophyceae | Fucales | Fucaceae | Fucus vesiculosus |
| | Excavata | Euglenozoa (Euglénides) | Euglenophyceae | Eutreptiales | Eutreptiaceae | Eutreptiella gymnastica |
| | Plantae | Charophyta (Charophytes) | Charophyceae | Charales | Characeae | Chara sp. |
| | | Chlorophyta (Algues vertes) | Trebouxiophyceae | Chlorellales | Chlorellaceae | Chlorella vulgaris |
| | | Rhodophyta (Algues rouges) | Florideophyceae | Palmariales | Palmariaceae | Palmaria palmata |

**Tableau 2 : Tableau récapitulatif des séquences selon la présente demande**

| **Numéro de la séquence** | **Précisions des espèces ou Divers** | **Séquences** |
|---|---|---|
| SEQ ID NO: 1 | Cible sur le gène LSU-END | GATGGTTCGATTAGTCTTTCGCCCCTATAC |
| SEQ ID NO : 2 | Cible sur le gène LSU-END | CTCGACCTATTCTCAAACTTTAAATG |
| SEQ ID NO: 3 | Régions des amorces LSU-END | GTATAGGGGCGAAAGACTAATCGAACCATC |
| SEQ ID NO : 4 | Régions des amorces LSU-END | CATTTAAAGTTTGAGAATAGGTCGAG |
| SEQ ID NO: 5 | Cible sur le gène LSU | TAGACTCCTTGGTCCGTGTTTCAAGACGGG |
| SEQ ID NO: 6 | Cible sur le gène LSU | GACCCGAAAGATGGTGAACTATGC |
| SEQ ID NO: 7 | Régions des amorces LSU | CCCGTCTTGAAACACGGACCAAGGAGTCTA |
| SEQ ID NO : 8 | Régions des amorces LSU | GCATAGTTCACCATCTTTCGGGTC |
| SEQ ID NO: 9 | *Fucus vesiculosus* | ATGCAAAGATCTWWRRAAMKAGWGWGAAAGAGTGCCTGAAATTGCTGAAAGGAAGC GAAGGATTATCAGTGTCTGGCGGTTCCGCTTCTCCTGCGTTCCTCGGAACGCTGGATCGC GTTTCCGTCGTGGTCAGTGCCGGCTAGCTTTGCGGGAGCCTGCGTCACGGGAGGTACGTC TCCTCGGAGATGGAATGCCGTGGCGTGTCGTCGCGAGGCTGGCCGGTCCTGACGAAATGG TATTCTTCACCCCGTCTTGAAACACGGACCAAGGAGTCTAACATGCCCGCGAGTACAGGG GCGAAAAACCCCCGTGCGCAGTGAAAGTGAATGCAGGTGGGAAGCGCAAGCGGCACCAT CGACCGACCATGTCTCTTCGGGGGCGAGGTTTGAGTGTGAGCGTGCATGTTGGGACCCGA AAGATGGTGAACTATGCCTGAATAGGGCGAAGCCAGGGGAAACTCTGGTGGAGGCTCGT AGCGATTCTGACGTGCAAATCGATCGTGAGATTTGGGTATAGGGGCGAAAGACTAATCGA ACCATCTAGTAGCTGGTTCCCTCCGAAATTCCCTCAGGATAGCTGCAATCGTTGTTCAGTTT |
| | | TATCA |
| SEQ ID NO : 10 | *Fucus vesiculosus* | AAGGAGTCTAGCACTACTGCGAGTCGAAAGGTTACTAAACCTAGAGGCAC AGTGAAAGCGAATATTGGGAAATCGAAAACAGATTAGGGAGGTGTTGCGA CTTTGTCAAAATATCCTACCCAGAGCAGTTGTGCTGGGA |
| SEQ ID NO : 11 | *Palmaria palmata* | AAGGAGTCTAACATGCCCGCGAGTACAGGGGTGGCAAACCCCCGTGCGCA GTGAAAGTGAATGCAGGTGGGACGCCTTCCTTTTAAAGGGAGGCTGCACC ATCGACCGACCGTGGCCCTCCGGGGTCGAGGTTTGAGTGTGAGCGCGTATG TTGGGA |
| SEQ ID NO : 12 | *Arthrospira platensis* | GCTGTGAAGAATTCCTTGTGGGGGATTCTGAGCCATCGGTGAGATACCACTCT GGCGAAGCTAGAATTCTAACCTTTTCCCGTTATCCGGGAAAGAGACAGTTTCA GGGGGGC |
| SEQ ID NO : 13 | *Arthrospira platensis* | GCTGTGAAGAATTCCTTGTGGGGGATTCTGAGCCATCGGTGAGATACCACTCT GGCGAGGCTAGAATTCTAACCTTTTCCCGTTATCCGGGAAAGAGACAGTTTCA GGGGGGC |
| SEQ ID NO : 14 | *Eutreptiella gymnastica* | GCTTTGAAGAATTCTTTCGGGAATATCTGAGCTGTTAGTGAGATACCATTCTAG GAGAGTTAGAAATCTAAAGACAGTTCTTGAATCAGAATGTTTGACAGTTTCAG GCGGGC |
| SEQ ID NO : 15 | *Eutreptiella gymnastica* | GCTTTGAAGAATTCTTTCGGGAATATCTGAGCTGTTAGTGAGATACCATTCTGG GAGAGTTAGAAATCTAAAGACAGTTCTTGAATCAGAATGTTTGACAGTTTCAG GCGGGC |
| SEQ ID NO : 16 | *Chara sp.* | TAGTAGCTGGTTCCCTCCGAAGTTTCCCTCAGGATAGCTGGAGCTGTTGAACAG TTTTATCGGGTAAAGCGAATGATTAGAGGCCTCGGGGGTGAAACACCC |
| SEQ ID NO : 17 | *Chlorella vulgaris* | TAGTAGCTGGTTCCCTCCGAAGTTTCCCCCAGGATAGCTGGAGCTCGTTTCAGTC GACTCGGGTAAAGCGAATGATTAGGCGCTCAGGGGATGAAACATCC |
| SEQ ID NO : 18 | *Fucus vesiculosus* | TAGTAGCTGGTTCCCTCCGAAATTTCCCTCAGGATAGCTGCAATCGTTGTTCAGT TTTATCAGGTAAAGCGAATGATTAGAGGCTGCGGGGGAGTAACTCCC |
| SEQ ID NO : 19 | *Eutreptiella gymnastica* | TAGTAGCTGGTTCCCTCCGAAGTTTCCCTCAGGATAGCTGGAGTTGAACAGTTTT ATCAGGTAAAGCGAATGATTAGAGGAATCGGGGGCGTGTTGTTC |
| SEQ ID NO : 20 | Amorce LSU | CCCGTCTTGAAACACGGACC |
| SEQ ID NO : 21 | Amorce LSU | CCCGTCTTGAAACACGGA |
| SEQ ID NO : 22 | Amorce LSU | CCCGTCTTGAAACACGGAC |
| SEQ ID NO : 23 | Amorce LSU | CCCGTCTTGAAACACGGACCA |
| SEQ ID NO : 24 | Amorce LSU | CCCGTCTTGAAACACGGACCAA |
| SEQ ID NO : 25 | Amorce LSU | CCCGTCTTGAAACACGGACCAAG |
| SEQ ID NO : 26 | Amorce LSU | CCCGTCTTGAAACACGGACCAAGG |
| SEQ ID NO : 27 | Amorce LSU | CACGGACCAAGGAGTCTA |
| SEQ ID NO : 28 | Amorce LSU | TTGAAACACGGACCAAGGAGTCTA |
| SEQ ID NO : 29 | Amorce LSU | AACACGGACCAAGGAGTCT |
| SEQ ID NO : 30 | Amorce LSU | AGTTCACCATCTTTCGGG |
| SEQ ID NO : 31 | Amorce LSU | GCATAGTTCACCATCTTT |
| SEQ ID NO : 32 | Amorce LSU | GCATAGTTCACCATCTTTC |
| SEQ ID NO : 33 | Amorce LSU | GCATAGTTCACCATCTTTCG |
| SEQ ID NO : 34 | Amorce LSU | GCATAGTTCACCATCTTTCGG |
| SEQ ID NO : 35 | Amorce LSU | GCATAGTTCACCATCTTTCGGG |
| SEQ ID NO : 36 | Amorce LSU | GCATAGTTCACCATCTTTCGGGT |
| SEQ ID NO : 37 | Amorce LSU | GCATAGTTCACCATCTTTCGGGTC |
| SEQ ID NO : 38 | Amorce LSU | TTCACCATCTTTCGGGTC |
| SEQ ID NO : 39 | Amorce LSU | CATAGTTCACCATCTTTCGG |
| SEQ ID NO : 40 | Amorce LSU | AMAAGTACCRYGAGGGAAAG |
| SEQ ID NO : 41 | Amorce LSU | SCWCTAATCATTCGCTTTACC |
| SEQ ID NO : 42 | Amorce LSU-END | GAAAGACTAATCGAACCATC |
| SEQ ID NO : 43 | Amorce LSU-END | GTATAGGGGCGAAAGACTAATCGA |
| SEQ ID NO : 44 | Amorce LSU-END | GTATAGGGGCGAAAGACTAATCG |
| SEQ ID NO : 45 | Amorce LSU-END | GTATAGGGGCGAAAGACTAATC |
| SEQ ID NO : 46 | Amorce LSU-END | GTATAGGGGCGAAAGACTAAT |
| SEQ ID NO : 47 | Amorce LSU-END | GTATAGGGGCGAAAGACTA |
| SEQ ID NO : 48 | Amorce LSU-END | GTATAGGGGCGAAAGACT |
| SEQ ID NO : 49 | Amorce LSU-END | GTATAGGGGCGAAAGACTAA |
| SEQ ID NO : 50 | Amorce LSU-END | TATAGGGGCGAAAGACTAAT |
| SEQ ID NO : 51 | Amorce LSU-END | ATAGGGGCGAAAGACTAATC |
| SEQ ID NO : 52 | Amorce LSU-END | TAGGGGCGAAAGACTAATCG |
| SEQ ID NO : 53 | Amorce LSU-END | AGGGGCGAAAGACTAATCGA |
| SEQ ID NO : 54 | Amorce LSU-END | GGGCGAAAGACTAATCGAAC |
| SEQ ID NO : 55 | Amorce LSU-END | GGCGAAAGACTAATCGAACC |
| SEQ ID NO : 56 | Amorce LSU-END | GCGAAAGACTAATCGAACCA |
| SEQ ID NO : 57 | Amorce LSU-END | CGAAAGACTAATCGAACCAT |
| SEQ ID NO : 58 | Amorce LSU-END | AAAGTTTGAGAATAGGTCGA |
| SEQ ID NO : 59 | Amorce LSU-END | CATTTAAAGTTTGAGAATAGGTCG |
| SEQ ID NO : 60 | Amorce LSU-END | CATTTAAAGTTTGAGAATAGGTC |
| SEQ ID NO : 61 | Amorce LSU-END | CATTTAAAGTTTGAGAATAGGT |
| SEQ ID NO : 62 | Amorce LSU-END | CATTTAAAGTTTGAGAATAGG |
| SEQ ID NO : 63 | Amorce LSU-END | CATTTAAAGTTTGAGAATA |
| SEQ ID NO : 64 | Amorce LSU-END | CATTTAAAGTTTGAGAAT |
| SEQ ID NO : 65 | Amorce LSU-END | CATTTAAAGTTTGAGAATAG |
| SEQ ID NO : 66 | Amorce LSU-END | ATTTAAAGTTTGAGAATAGG |
| SEQ ID NO : 67 | Amorce LSU-END | TTTAAAGTTTGAGAATAGGT |
| SEQ ID NO : 68 | Amorce LSU-END | TTAAAGTTTGAGAATAGGTC |
| SEQ ID NO : 69 | Amorce LSU-END | TAAAGTTTGAGAATAGGTCG |
| SEQ ID NO : 70 | Amorce LSU-END | AAGTTTGAGAATAGGTCGAG |

**Tableau 3: Positions d'intérêt sur les gènes LSU et LSU-END**

| **Espèce :** | **Positions sur le gène *LSU* :** | **Positions sur le gène *LSU-END* :** |
|---|---|---|
| *Chara sp.* | 323 à 465 | 595 à 698 |
| *Chlorella vulgaris* | | |
| *Eutreptiella gymnastica* | | |
| *Fucus vesiculosus* | 267 à 410 | *541 à 643* |
| *Palmaria palmata* | 235 à 364 | |
| *Athrospira platensis* | | |
| *Symbiodinium microadriaticum* | 312 à 444 | |

La présente demande sera mieux illustrée par les exemples suivants. Les exemples ci-après visent à éclaircir la présente demande et illustrer des modes de réalisation avantageux, mais en aucun cas visent à restreindre la portée de la présente demande.

### Exemple 1 : Méthode avec des produits à base d'algues

### Extraction d'ADN :

### Sur produits à base d'algue :

L'ADN a été extrait des produits à base d'algue en utilisant un protocole adapté du kit DNeasy Blood and Tissue kit (Qiagen).

### Amplification PCR :

### Sur produits à base d'algue :

Les ADNs extraits des produits à base d'algue ont été amplifiés pour deux marqueurs génétiques à l'aide des amorces correspondantes (Tableau 4). Une étiquette de 8 bases est ajoutée du côté 5' de l'amorce afin de créer un système de « multiplexage » et de permettre l'assignation des séquences à leurs échantillons respectifs.

**Tableau 4 : Liste des amorces utilisées au cours de l'étude sur les produits à base d'algue**

| **Marqueur :** | **Amorces :** | | | | **Température d'hybridation :** |
|---|---|---|---|---|---|
| | **Amorce sens :** | | **Amorce antisens :** | | |
| LSU | LSU_f1 | SEQ ID NO : 20 | LSU_r1 | SEQ ID NO : 30 | 55°C |
| LSU-END | LSU-END_f1 | SEQ ID NO : 42 | LSU-END_r1 | SEQ ID NO : 58 | 60°C |

Le mélange réactionnel utilisé pour l'amplification a été préparé comme suit : tampon : 1X ; MgCl2 : 2 mM ; dNTP : 0,2 mM chacun; amorces: 0,2 µM chacune; BSA: 0,16 mg.µL⁻¹; Taq Polymérase (Hot start FIREPol ; Solis): 2 unités; 3 µL d'ADN; pour un volume final de 25 µL. Il a ensuite été amplifié sur un thermocycleur (T100, Biorad) en utilisant le programme défini en Tableau 5.

**Tableau 5 : Programme d'amplification des marqueurs génétiques sur produits à base d'algue**

| **Programme PCR** | | |
|---|---|---|
| Dénaturation initiale | 95°C | 10 min |
| Nombre de cycle | 50 | |
| Dénaturation | 95°C | 30 s |
| Hybridation | voir tableau 4 | 30 s |
| Élongation | 72°C | 1 min |
| Élongation finale | 72°C | 7 min |
| Fin | 10°C | |

### Séquençage:

### Sur produits à base d'algue:

Les produits d'amplification ont été analysés par électrophorèse capillaire (QIAxcel DNA Screening kit, Qiagen). Les marqueurs ayant été correctement amplifiés ont ensuite été purifiés avec le kit NucleoSpin Gel and PCR Clean-up (Macherey-Nagel) et dosés au spectrophotomètre SimpliNano 4285 v2.0.0 (GE HealthCare). Les échantillons ont ensuite été poolés et normalisés. La librairie à séquencer est préparée à partir du Truseq PCR-free library préparation Kit (Illumina). Le séquençage en paired-end 2x150 bp a été réalisé sur séquenceur nouvelle génération HiSeq (Illumina).

### Exemple 2 : Démarche avec les marqueurs courts

| **Typologie de produit :** | **Extraction :** | **Marqueurs à utiliser :** | **Type de séquençage :** | **Résultats attendus :** |
|---|---|---|---|---|
| **Algues en mélange** | Extraction avec le « protocole produit » | Amplification du marqueur LSU-END court (possibilité de rajouter le marqueur LSU court) puis électrophorèse et purification | Séquençage NGS | Détermination de l'embranchement, de la famille, voire le genre |
| **Produits à base d'algues** | Extraction avec le « protocole produit » | Amplification du marqueur LSU-END court (possibilité de rajouter le marqueur LSU court) puis électrophorèse et purification | Séquençage NGS | Détermination de l'embranchement de la famille, voire le genre |
| **Produits à base d'algues et de plantes** | Extraction avec le « protocole produit » | Amplification du marqueur LSU-END court (possibilité de rajouter le marqueur LSU court) puis électrophorèse et purification | Séquençage NGS | Détermination de l'embranchement de la famille, voire le genre |

### Exemple 3 : Exemple avec des produits à base d'algue

### Extraction d'ADN :

L'ADN des 13 produits à base d'algues a été extrait en utilisant un protocole adapté du kit DNeasy Blood and Tissue kit (Qiagen).

### Amplification PCR :

Les ADNs extraits des produits à base d'algues ont été amplifiés pour deux marqueurs génétiques à l'aide des amorces correspondantes (Tableau 1). Une étiquette de 8 bases nucléotidiques est ajoutée du coté 5' de l'amorce afin de créer un système de « multiplexage » et de permettre l'assignation des séquences à leurs échantillons respectifs (COISSAC, Eric, RIAZ, Tiayyba et PUILLANDRE, Nicolas. Bioinformatic Challenges for DNA metabarcoding of plantes and animals. Molecular Ecology, 2012, vol. 21, pp. 1834-1847).

**Tableau 6 : Liste des amorces utilisées au cours de l'étude sur les produits à base d'algue**

| Marqueur | Amorces | | | | Température d'hybridation |
|---|---|---|---|---|---|
| | Amorces sens | | Amorces antisens | | |
| LSU | LSU_f1 | SEQ ID NO: 20 | LSU_r1 | SEQ ID NO: 30 | 55°C |
| LSU-END | LSU-END_f1 | SEQ ID NO: 42 | LSU-END_r1 | SEQ ID NO: 58 | 60°C |

Le mélange réactionnel utilisé pour l'amplification a été préparé comme suit : tampon : 1X ; MgCl2 : 2 mM ; dNTP : 0,2 mM chacun ; amorces : 0,2 µM chacune ; BSA : 0,16 mg.µL⁻¹ ; Taq Polymérase (Hot start FIREPo1 ; Solis) : 2 unités ; 3 µL d'ADN ; pour un volume final de 25 µL. L'amplification est ensuite réalisée sur un thermocycleur (T100, Biorad) en utilisant le programme défini en Tableau 2.

**Tableau 7 : Programme d'amplification des marqueurs génétiques sur produits à base d'algue**

| Programme PCR | | |
|---|---|---|
| Dénaturation initiale | 95°C | 10min |
| Nombre de cycle | 50 | |
| Dénaturation | 95°C | 30 s |
| Hybridation | voir tableau 1 | 30 s |
| Élongation | 72°C | 1 min |
| Élongation finale | 72°C | 7 min |
| Fin | 10°C | |

### Séquençage sur produits à base d'algues :

Les produits d'amplification ont été analysés par électrophorèse capillaire (QIAxcel, Qiagen). Les marqueurs amplifiés ont ensuite été purifiés avec le kit NucleoSpin Gel and PCR Clean-up (Macherey-Nagel) et dosés par spectrophotométrie (SimpliNano, GE Health care). Les échantillons ont ensuite été poolés et normalisés. La librairie à séquencer est préparée à partir du Truseq PCR-free library préparation Kit (Illumina). Le séquençage des amplicons a été réalisé sur un système MiSeq (Illumina).

### Résultats du séquençage:

Les résultats du séquençage nouvelle génération (NGS) sont constitués des différentes séquences génétiques amplifiées dans le produit d'extraction de l'échantillon analysé (Tableau 3). Ces séquences d'ADN proviennent des algues entrant dans la composition initiale du produit et/ou de tissus ou pollens de plantes qui arrivent pendant ou à la fin du processus de fabrication du produit ou lors des transferts et des échantillonnages.

Il est à noter que dans les colonnes `Composition LSU', ne sont reportés que les noms de familles, de genres ou d'espèces d'algues présentes dans l'échantillon d'après la composition annoncée. Par ailleurs, pour chaque famille, genre ou espèce d'algues présentes dans le Tableau 3, plusieurs séquences peuvent avoir été identifiées. Enfin, entre parenthèses sont reportés les numéros d'accessions des séquences correspondant à l'espèce assignée. Pour les assignations à la famille ou au genre, comme un grand nombre de numéros d'accession correspondent à l'assignation, les numéros d'accession ne sont pas reportés dans le tableau.

**Tableau 8 : Résultats du séquençage des produits à base d'algues pour les marqueurs LSU et LSU-END. Le caractère Θ signifie que l'analyse n'a pas été effectuée**

| **Nom du produit à base d'algue** | **Marque du vendeur** | **Composition annoncée en algues** | **Composition *LSU* (N° accession)** | **Composition *LSU-*END (N° accession)** |
|---|---|---|---|---|
| Rillettes de maquereaux aux fines algues | La Compagnie Bretonne du Poisson | farine de kombu (*Laminaria digita*), algues réhydratées Dulse (*Palmaria palmata*), Nori (*Porphyra*), Laitue de mer (*Ulva lactuca*) | Θ | Chlorophyta Pyropia haitanensis (JN104581) |
| Bezhin (équilibre minéral) | Thalado, Le comptoir des Algues | poudre de Lithotamne, poudre de Spiruline, poudre d'ulves | Θ | *Chlorophyta* |
| Agar-agar (stick) | Thalado, Le comptoir des Algues | extraits d'algues rouges | Gelidium | Θ |

### Exemple 5: Détermination de l'embranchement, l'ordre, la famille, le genre ou l'espèce d'une algue

- L'algue à analyser avec le marqueur *LSU-END* est une algue verte. Dans la base de données de référence issue de Genbank, les séquences de *Chlorella vulgaris* et de *Ulva lactuca* sont identiques pour le marqueur *LSU-END.* L'assignation de l'algue à analyser se fera donc conjointement à *Chlorella vulgaris* et de *Ulva lactuca.* L'algue *Chlorella vulgaris* appartient à l'ordre des chlorellales et *Ulva lactuca* appartient à l'ordre des Ulvales. Les deux algues appartiennent à l'embranchement *Chlorophyta.* L'assignation de l'algue à analyser se fera donc à l'embranchement *Chlorophyta.*
- L'algue à analyser avec le marqueur *LSU-END* est une algue rouge. Dans la base de données de référence issue de Genbank^{®}, la séquence de chaque espèce de *Pyropia* est différente pour *LSU-END.* L'assignation de l'algue à analyser se fera donc à l'espèce *Pyropia haitanensis.*
- L'algue à analyser avec le marqueur *LSU* est une algue rouge. Dans la base de données de référence issue de Genbank^{®}, les séquences de chaque espèce de *Gelidium spinosum* et *Gelidium pulchellum* sont identiques pour *LSU*. L'assignation de l'algue à analyser se fera donc conjointement à *Gelidium spinosum et Gelidium pulchellum* avec le même pourcentage d'identité. Les deux appartiennent au genre *Gelidium.* L'assignation de l'algue à analyser se fera donc au genre *Gelidium.*

## Revendications

1. Utilisation d'une paire d'amorces d'amplification pour la détection et/ou l'identification d'une algue dans un échantillon susceptible d'en contenir, par amplification d'au moins une région variable du gène *LSU* codant pour la grande sous-unité d'un ribosome,
dans laquelle ladite région variable amplifiée du gène LSU d'une algue a une taille inférieure ou égale à 220 paires de bases, ladite région variable constituant un marqueur de détection et/ou d'identification d'une algue,
dans laquelle:
- une première paire d'amorces d'amplification consiste en un premier oligonucléotide qui s'hybride à une séquence de 15 à 30 nucléotides contigus, de préférence de 18 à 24 nucléotides contigus, choisie dans la séquence de SEQ ID NO: 1 du gène *LSU* d'une algue et un deuxième oligonucléotide qui s'hybride à une séquence de 15 à 30 nucléotides contigus, de préférence de 18 à 24 nucléotides contigus, choisie dans la séquence de SEQ ID NO: 2 du gène *LSU* d'une algue, ou
- une deuxième paire d'amorces d'amplification consiste en un premier oligonucléotide qui s'hybride à une séquence de 15 à 30 nucléotides contigus, de préférence de 18 à 24 nucléotides contigus choisie dans la séquence de SEQ ID NO: 5 du gène *LSU* d'une algue et le deuxième oligonucléotide s'hybride à une séquence de 15 à 30 nucléotides contigus, de préférence de 18 à 24 nucléotides contigus choisie dans la séquence de SEQ ID NO: 6 du gène *LSU* d'une algue.

2. Utilisation d'une paire d'amorces d'amplification selon la revendication 1, dans laquelle ledit premier oligonucléotide ou ledit deuxième oligonucléotide de la première ou de la deuxième paire d'amorces d'amplification s'hybride à sa séquence cible respective choisie parmi les séquences SEQ ID NO: 1, SEQ ID NO:2, SEQ ID NO: 5, et SEQ ID NO: 6 du gène *LSU* d'une algue, à une température comprise de 40 à 65°C, et plus particulièrement de 50-60°C et une concentration saline comprise de 0, 001 à 0,5 M, et plus particulièrement de 0,05 à 0,1 M, et encore plus particulièrement de à 0,001 à 0,005M.

3. Utilisation d'une paire d'amorces d'amplification selon la revendication 1, dans laquelle le premier oligonucléotide de la première paire d'amorces d'amplification a une séquence de 15 à 30 nucléotides contigus, de préférence de 18 à 24 nucléotides contigus choisie dans la séquence de SEQ ID NO: 3 et le deuxième oligonucléotide a une séquence de 15 à 30 nucléotides contigus, de préférence de 18 à 24 nucléotides contigus choisie dans la séquence de SEQ ID NO: 4,
le premier oligonucléotide de la deuxième paire d'amorces d'amplification a une séquence de 15 à 30 nucléotides contigus, de préférence de 18 à 24 nucléotides contigus choisie dans la séquence de SEQ ID NO: 7 et le deuxième oligonucléotide a une séquence de 15 à 30 nucléotides contigus, de préférence de 18 à 24 nucléotides contigus choisie dans la séquence de SEQ ID NO: 8,
pour la détection et/ou l'identification d'une algue dans un échantillon susceptible d'en contenir, par amplification d'au moins une région variable du gène *LSU* d'une algue, ladite région variable constituant un marqueur de détection et/ou d'identification d'une algue, et dans laquelle ladite algue appartient à l'un des embranchements suivants: *Glaucophyta, Cyanobacteria, Dinophyta, Ochrophyta, Euglenozoa, Charophyta, Chlorophyta, ou Rhodophyta.*

4. Utilisation d'une paire d'amorces d'amplification selon l'une quelconque des revendications précédentes, dans laquelle ladite région variable du gène LSU d'une algue est représentée par une séquence choisie dans le groupe constitué par:, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, ou une séquence ayant un pourcentage d'identité d'au moins 80% avec ladite séquence et notamment 95% et plus particulièrement 98% ou 99%.

5. Paire d'amorces d'amplification ayant une taille comprise de 15 à 45 nucléotides, notamment de 15 à 30 nucléotides contigus, de préférence de 18 à 24 nucléotides contigus, dans laquelle une première paire d'amorces d'amplification comprend ou est constituée par
- SEQ ID NO: 1, ou une séquence ayant un pourcentage d'identité d'au moins 80%, 81%, 82%, 83%, 84%, 85%, 86%, 86%, 88%, 89%, 90%, 91%, 92%, 93%, 94%, 95%, 96%, 97%, 98%, 99% avec ladite séquence de SEQ ID NO: 1, et
- SEQ ID NO: 2, ou une séquence ayant un pourcentage d'identité d'au moins 80%, 81%, 82%, 83%, 84%, 85%, 86%, 86%, 88%, 89%, 90%, 91%, 92%, 93%, 94%, 95%, 96%, 97%, 98%, 99% avec ladite séquence de SEQ ID NO: 2, dans laquelle une seconde paire d'amorces d'amplification comprend ou est constituée par
- SEQ ID NO: 5, ou une séquence ayant un pourcentage d'identité d'au moins 80%, 81%, 82%, 83%, 84%, 85%, 86%, 86%, 88%, 89%, 90%, 91%, 92%, 93%, 94%, 95%, 96%, 97%, 98%, 99% avec ladite séquence de SEQ ID NO: 5, et
- SEQ ID NO: 6, ou une séquence ayant un pourcentage d'identité d'au moins 80%, 81%, 82%, 83%, 84%, 85%, 86%, 86%, 88%, 89%, 90%, 91%, 92%, 93%, 94%, 95%, 96%, 97%, 98%, 99% avec ladite séquence de SEQ ID NO: 6,
permettant d'amplifier un fragment d'une taille inférieure ou égale à 220 paires de bases d'une région variable du gène LSU codant pour la grande sous-unité d'un ribosome, d'une algue.

6. Paire d'amorces d'amplification selon la revendication 5, ladite amorce d'amplification ayant une taille comprise de 15 à 30 nucléotides contigus, de préférence de 18 à 24 nucléotides contigus,
dans laquelle une première paire d'amorces d'amplification est choisie dans la séquence de SEQ ID NO : 7 et SEQ ID NO : 8 comprenant ou étant constituée par une séquence choisie parmi SEQ ID NO : 20, SEQ ID NO : 21, SEQ ID NO : 22, SEQ ID NO : 23, SEQ ID NO : 24, SEQ ID NO : 25, SEQ ID NO : 26, SEQ ID NO : 27, SEQ ID NO :28, SEQ ID NO : 29, SEQ ID NO : 30, SEQ ID NO : 31, SEQ ID NO : 32, SEQ ID NO : 33, SEQ ID NO : 34, SEQ ID NO : 35, SEQ ID NO : 36, SEQ ID NO : 37, SEQ ID NO : 38, SEQ ID NO : 39, SEQ ID NO : 40, SEQ ID NO : 41,
dans laquelle une seconde paire d'amorces d'amplification est choisie dans la séquence de SEQ ID NO : 3 et SEQ ID NO : 4 comprenant ou étant constituée par une séquence choisie parmi SEQ ID NO : 42 , SEQ ID NO : 43, SEQ ID NO : 44, SEQ ID NO : 45, SEQ ID NO : 46, SEQ ID NO : 47, SEQ ID NO : 48, SEQ ID NO : 49, SEQ ID NO : 50, SEQ ID NO : 51, SEQ ID NO : 52, SEQ ID NO : 53, SEQ ID NO : 54, SEQ ID NO : 55, SEQ ID NO : 56, SEQ ID NO : 57, SEQ ID NO : 58, SEQ ID NO : 59, SEQ ID NO : 60, SEQ ID NO : 61, SEQ ID NO : 62, SEQ ID NO : 63, SEQ ID NO : 64, SEQ ID NO : 65, SEQ ID NO : 66, SEQ ID NO: 67, SEQ ID NO: 68, SEQ ID NO: 69, SEQ ID NO: 70, ou une séquence ayant un pourcentage d'identité d'au moins 81%, 82%, 83%, 84%, 85%, 86%, 86%, 88%, 89%, 90%, 91%, 92%, 93%, 94%, 95%, 96%, 97%, 98%, 99% ou 100% avec lesdites séquences.

7. Marqueur de détection et/ou d'identification d'une algue ayant une taille inférieure ou égale à 220 nucléotides d'une région variable amplifiée du gène LSU codant pour la grande sous-unité d'un ribosome, d'une algue , notamment inférieure ou égale à 190, 180 et notamment 178 nucléotides, ledit marqueur comprenant ou étant constitué par une séquence ayant un pourcentage d'identité d'au moins 98%, 99%, ou 100% avec une séquence choisie parmi, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, et SEQ ID NO: 19.

8. Procédé d'amplification d'au moins une région variable du gène *LSU* d'une algue, ladite région variable amplifiée du gène LSU codant pour la grande sous-unité d'un ribosome d'une taille inférieure ou égale à 220 paires de bases constituant un marqueur de détection et/ou d'identification d'une algue, comprenant ou étant constitué des étapes suivantes:
a) une étape d'extraction du matériel nucléique d'un produit ou échantillon susceptible de contenir une algue,
b) une étape d'amplification de ladite région variable du gène *LSU* d'une algue, en utilisant
- une première paire d'amorces d'amplification consiste en un premier oligonucléotide qui s'hybride à une séquence de 15 à 30 nucléotides contigus, de préférence de 18 à 24 nucléotides contigus choisie dans la séquence de SEQ ID NO: 1 du gène *LSU* d'une algue et un deuxième oligonucléotide qui s'hybride à une séquence de 15 à 30 nucléotides contigus, de préférence de 18 à 24 nucléotides contigus choisie dans la séquence de SEQ ID NO: 2 du gène *LSU* d'une algue, ou
- une deuxième paire d'amorces d'amplification consiste en un premier oligonucléotide qui s'hybride à une séquence de 15 à 30 nucléotides contigus, de préférence de 18 à 24 nucléotides contigus choisie dans la séquence de SEQ ID NO: 5 du gène *LSU* d'une algue et le deuxième oligonucléotide s'hybride à une séquence de 15 à 30 nucléotides contigus, de préférence de 18 à 24 nucléotides contigus choisie dans la séquence de SEQ ID NO: 6 du gène *LSU* d'une algue,
c) une étape de détermination ou d'identification de la présence d'une algue dans ledit produit ou échantillon par la détection d'au moins une région variable amplifiée du gène *LSU* d'une algue.

9. Procédé d'amplification selon la revendication 8, dans lequel ledit procédé comprend l'obtention d'au moins une région variable amplifiée du gène *LSU* d'une algue, notamment une étape de détection de ladite obtention d'au moins une région variable amplifiée du gène *LSU* d'une algue afin d'obtenir une région variable amplifiée et détectée du gène *LSU* d'une algue, et dans lequel ledit procédé comprend de préférence une étape d'identification d'une algue par séquençage de ladite région variable amplifiée et détectée du gène *LSU* d'une algue afin d'obtenir une région variable amplifiée séquencée.

## Patentansprüche

1. Verwendung eines Amplifikationsprimerpaares zum Nachweis und/oder zur Identifizierung einer Alge in einer Probe, die möglicherweise eine solche enthält, durch Amplifikation mindestens einer variablen Region des *LSU-Gens,* das für die große Untereinheit eines Ribosoms kodiert,
wobei die amplifizierte variable Region des LSU-Gens einer Alge eine Größe von kleiner oder gleich 220 Basenpaaren aufweist,
wobei die variable Region einen Marker zum Nachweis und/oder zur Identifizierung einer Alge darstellt, wobei:
- ein erstes Amplifikationsprimerpaar aus einem ersten Oligonukleotid, das mit einer Sequenz von 15 bis 30 aufeinanderfolgenden Nukleotiden, vorzugsweise 18 bis 24 aufeinanderfolgenden Nukleotiden, ausgewählt aus der Sequenz von SEQ ID NO: 1 des *LSU*-Gens einer Alge, hybridisiert, und einem zweiten Oligonukleotid, das mit einer Sequenz von 15 bis 30 aufeinanderfolgenden Nukleotiden, vorzugsweise 18 bis 24 aufeinanderfolgenden Nukleotiden, ausgewählt aus der Sequenz von SEQ ID NO: 2 des *LSU*-Gens einer Alge, hybridisiert besteht, oder
- ein zweites Amplifikationsprimerpaar aus einem ersten Oligonukleotid, das mit einer Sequenz von 15 bis 30 aufeinanderfolgenden Nukleotiden, vorzugsweise 18 bis 24 aufeinanderfolgenden Nukleotiden, ausgewählt aus der Sequenz von SEQ ID NO: 5 des *LSU*-Gens einer Alge, hybridisiert, und das zweite Oligonukleotid mit einer Sequenz von 15 bis 30 aufeinanderfolgenden Nukleotiden, vorzugsweise 18 bis 24 aufeinanderfolgenden Nukleotiden, ausgewählt aus der Sequenz von SEQ ID NO: 6 des *LSU-Gens* einer Alge, hybridisiert besteht.

2. Verwendung eines Amplifikationsprimerpaars nach Anspruch 1, wobei das erste Oligonukleotid oder das zweite Oligonukleotid des ersten oder zweiten Amplifikationsprimerpaars mit seiner jeweiligen Zielsequenz, ausgewählt aus den Sequenzen SEQ ID NO: 1, SEQ ID NO: 2, SEQ ID NO: 5 und SEQ ID NO: 6 des *LSU-Gens* einer Alge, bei einer Temperatur von 40 bis 65 °C, insbesondere 50 bis 60 °C, und einer Salzkonzentration im Bereich von 0,001 bis 0,5 M, bevorzugt 0,05 bis 0,1 M, und noch stärker bevorzugt 0,001 bis 0,005 M hybridisiert.

3. Verwendung eines Amplifikationsprimerpaars nach Anspruch 1, wobei das erste Oligonukleotid des ersten Amplifikationsprimerpaars eine Sequenz von 15 bis 30 aufeinanderfolgenden Nukleotiden, vorzugsweise von 18 bis 24 aufeinanderfolgenden Nukleotiden, ausgewählt aus der Sequenz von SEQ ID NO: 3, aufweist und das zweite Oligonukleotid eine Sequenz von 15 bis 30 aufeinanderfolgenden Nukleotiden, vorzugsweise von 18 bis 24 aufeinanderfolgenden Nukleotiden, ausgewählt aus der Sequenz von SEQ ID NO: 4, aufweist, das erste Oligonukleotid des zweiten Amplifikationsprimerpaars eine Sequenz von 15 bis 30 aufeinanderfolgenden Nukleotiden, vorzugsweise von 18 bis 24 aufeinanderfolgenden Nukleotiden, ausgewählt aus der Sequenz von SEQ ID NO: 7, aufweist und das zweite Oligonukleotid eine Sequenz von 15 bis 30 aufeinanderfolgenden Nukleotiden, vorzugsweise von 18 bis 24 aufeinanderfolgenden Nukleotiden, ausgewählt aus der Sequenz 5 von SEQ ID NO: 8, aufweist, zum Nachweis und/oder zur Identifizierung einer Alge in einer Probe, die möglicherweise eine solche enthält, durch Amplifikation mindestens einer variablen Region des *LSU-Gens* einer Alge, wobei die variable Region einen Marker zum Nachweis und/oder zur Identifizierung einer Alge darstellt, und wobei die Alge zu einer der folgenden Gruppen gehört: *Glaucophyta, Cyanobacteria, Dinophyta, Ochrophyta, Euglenozoa, Charophyta, Chlorophyta* oder *Rhodophyta.*

4. Verwendung eines Amplifikationsprimerpaares nach einem der vorhergehenden Ansprüche, wobei die variable Region des LSU-Gens einer Alge durch eine Sequenz dargestellt wird, ausgewählt aus der Gruppe bestehend aus: SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, oder einer Sequenz mit einer prozentualen Identität von mindestens 80 ö mit der Sequenz und bevorzugt 95 ö und noch stärker bevorzugt 98 ö oder 99 %.

5. Amplifikationsprimerpaar mit einer Größe im Bereich von 15 bis 45 Nukleotiden, insbesondere 15 bis 30 aufeinanderfolgenden Nukleotiden, vorzugsweise 18 bis 24 aufeinanderfolgenden Nukleotiden, wobei ein erstes Amplifikationsprimerpaar umfasst oder besteht aus:
- SEQ ID NO: 1 oder einer Sequenz mit einer prozentualen Identität von mindestens 80 %, 81 %, 82 %, 83 %, 84 %, 85 %, 86 %, 86 %, 88 %, 89 %, 90 %, 91 %, 92 %, 93 %, 94 %, 95 %, 96 %, 97 %, 98 %, 99 ö mit der Sequenz von SEQ ID NO: 1, und
- SEQ ID NO: 2 oder einer Sequenz mit einer prozentualen Identität von mindestens 80 %, 81 %, 82 %, 83 %, 84 %, 85 %, 86 %, 86 %, 88 %, 89 %, 90 %, 91 %, 92 %, 93 %, 94 %, 95 %, 96 %, 97 %, 98 %, 99 ö mit der Sequenz von SEQ ID NO: 2,
wobei ein zweites Amplifikationsprimerpaar umfasst oder besteht aus:
- SEQ ID NO: 5 oder einer Sequenz mit einer prozentualen Identität von mindestens 80 %, 81 %, 82 %, 83 %, 84 %, 85 %, 86 %, 86 %, 88 %, 89 %, 90 %, 91 %, 92 %, 93 %, 94 %, 95 %, 96 %, 97 %, 98 %, 99 ö mit der Sequenz von SEQ ID NO: 5, und
- SEQ ID Nr. 6 oder einer Sequenz mit einer prozentualen Identität von mindestens 80 %, 81 %, 82 %, 83 %, 84 %, 85 %, 86 %, 86 %, 88 %, 89 %, 90 %, 91 %, 92 %, 93 %, 94 %, 95 %, 96 %, 97 %, 98 %, 99 ö mit der Sequenz von SEQ ID Nr. 6,
die es ermöglicht, ein Fragment mit einer Größe von kleiner oder gleich 220 Basenpaaren aus einer variablen Region des LSU-Gens, das für die große Untereinheit eines Ribosoms kodiert, aus einer Alge zu amplifizieren.

6. Amplifikationsprimerpaar nach Anspruch 5, wobei der Amplifikationsprimer eine Größe im Bereich von 15 bis 30 aufeinanderfolgenden Nukleotiden, vorzugsweise 18 bis 24 aufeinanderfolgenden Nukleotiden, aufweist,
wobei ein erstes Amplifikationsprimerpaar ausgewählt ist aus der Sequenz von SEQ ID NO: 7 und SEQ ID NO: 8, umfassend oder bestehend aus einer Sequenz, ausgewählt aus SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO:39, SEQ ID NO:40, SEQ ID NO:41,
wobei ein zweites Amplifikationsprimerpaar ausgewählt ist aus der Sequenz von SEQ ID NO: 3 und SEQ ID NO: 4, umfassend oder bestehend aus einer Sequenz, ausgewählt aus SEQ ID NO: 42, SEQ ID NO: 43, SEQ ID NO: 44, SEQ ID NO: 45, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, oder einer Sequenz, die eine prozentuale Identität von mindestens 81 %, 82 %, 83 %, 84 %, 85 %, 86 %, 86 %, 88 %, 89 %, 90 %, 91 %, 92 %, 93 %, 94 %, 95 %, 96 %, 97 %, 98 %, 99 ö oder 100 ö mit diesen Sequenzen aufweist.

7. Marker zum Nachweis und/oder zur Identifizierung einer Alge mit einer Größe von kleiner oder gleich 220 Nukleotiden einer amplifizierten variablen Region des Gens LSU, das für die große Untereinheit eines Ribosoms einer Alge kodiert, insbesondere kleiner oder gleich 190, 180 und insbesondere 178 Nukleotiden, wobei der Marker eine Sequenz umfasst oder aus einer Sequenz besteht, die eine prozentuale Identität von mindestens 98 %, 99 ö oder 100 ö mit einer Sequenz, ausgewählt aus SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18 und SEQ ID NO: 19, aufweist.

8. Verfahren zur Amplifikation mindestens einer variablen Region des *LSU-Gens* einer Alge, wobei die amplifizierte variable Region des LSU-Gens für die große Untereinheit eines Ribosoms mit einer Größe von kleiner oder gleich 220 Basenpaaren kodiert, das einen Marker zum Nachweis und/oder zur Identifizierung einer Alge darstellt, umfassend oder bestehend aus den folgenden Schritten:
a) einem Schritt zum Extrahieren von Nuklearmaterial aus einem Produkt oder einer Probe, das/die möglicherweise eine Alge enthält,
b) einem Schritt zum Amplifizieren dieser variablen Region des *LSU-Gens* einer Alge unter Verwendung
- eines ersten Amplifikationsprimerpaars, das aus einem ersten Oligonukleotid, das mit einer Sequenz von 15 bis 30 aufeinanderfolgenden Nukleotiden, vorzugsweise 18 bis 24 aufeinanderfolgenden Nukleotiden, ausgewählt aus der Sequenz von SEQ ID NO: 1 des *LSU-Gens* einer Alge, hybridisiert, und einem zweiten Oligonukleotid, das mit einer Sequenz von 15 bis 30 aufeinanderfolgenden Nukleotiden, vorzugsweise 18 bis 24 aufeinanderfolgenden Nukleotiden, ausgewählt aus der Sequenz von SEQ ID NO: 2 des *LSU-Gens* einer Alge, hybridisiert, besteht, oder
- einem zweiten Amplifikationsprimerpaar, das aus einem ersten Oligonukleotid, das mit einer Sequenz von 15 bis 30 aufeinanderfolgenden Nukleotiden, vorzugsweise 18 bis 24 aufeinanderfolgenden Nukleotiden, ausgewählt aus der Sequenz von SEQ ID NO: 5 des *LSU-Gens* einer Alge, hybridisiert, und einem zweiten Oligonukleotid besteht, das einer Sequenz von 15 bis 30 aufeinanderfolgenden Nukleotiden, vorzugsweise 18 bis 24 aufeinanderfolgenden Nukleotiden, ausgewählt aus der Sequenz von SEQ ID NO: 6 des *LSU-*Gens einer Alge, hybridisiert besteht,
c) einem Schritt zum Bestimmen oder Identifizieren des Vorhandenseins einer Alge in dem Produkt oder der Probe durch den Nachweis von mindestens einer amplifizierten variablen Region des *LSU-Gens* einer Alge.

9. Amplifikationsverfahren nach Anspruch 8, wobei das Verfahren das Erhalten von mindestens einer amplifizierten variablen Region des *LSU-Gens* einer Alge umfasst, insbesondere einen Schritt zum Nachweisen des Erhalts von mindestens einer amplifizierten variablen Region des LSU-Gens einer Alge, um eine amplifizierte und nachgewiesene variable Region des *LSU-Gens* einer Alge zu erhalten, und wobei das Verfahren vorzugsweise einen Schritt zum Identifizieren einer Alge durch Sequenzieren der amplifizierten und nachgewiesenen variablen Region des *LSU-Gens* einer Alge umfasst, um eine sequenzierte amplifizierte variable Region zu erhalten.

## Claims

1. Use of a pair of amplification primers for the detection and/or identification of an alga in a sample likely to contain it, by amplification of at least one variable region of the *LSU* gene coding for the large subunit of a ribosome,
wherein said amplified variable region of the LSU gene of an alga has a size less than or equal to 220 base pairs, said variable region constituting a marker for the detection and/or identification of an alga,
wherein:
- a first pair of amplification primers consists of a first oligonucleotide that hybridizes to a sequence from 15 to 30 contiguous nucleotides, preferably from 18 to 24 contiguous nucleotides, chosen in the SEQ ID NO: 1 sequence of the *LSU* gene of an alga and a second oligonucleotide that hybridizes to a sequence from 15 to 30 contiguous nucleotides, preferably from 18 to 24 contiguous nucleotides, chosen in the SEQ ID NO: 2 sequence of the *LSU gene* of an alga, or
- a second pair of amplification primers consists of a first oligonucleotide that hybridizes to a sequence from 15 to 30 contiguous nucleotides, preferably from 18 to 24 contiguous nucleotides chosen in the sequence of SEQ ID NO:5 of the *LSU* gene of an alga and the second oligonucleotide hybridizes to a sequence from 15 to 30 contiguous nucleotides, preferably from 18 to 24 contiguous nucleotides chosen in the SEQ ID NO:6 sequence of the *LSU gene* of an alga.

2. The use of an amplification primer pair according to claim 1, wherein said first oligonucleotide or said second oligonucleotide of the first or second pair of amplification primers hybridizes to its respective target sequence chosen from the sequences SEQ ID NO: 1, SEQ ID NO:2, SEQ ID NO: 5, and SEQ ID NO: 6 of the *LSU* gene of an alga, at a temperature from 40 to 65°C, and more particularly from 50-60°C and a saline concentration from 0.001 to 0.5 M, and more particularly from 0.05 to 0.1 M, and even more particularly from 0.001 to 0.005M.

3. The use of a pair of amplification primers according to claim 1, wherein the first oligonucleotide of the first pair of amplification primers has a sequence from 15 to 30 contiguous nucleotides, preferably from 18 to 24 contiguous nucleotides chosen in the sequence of SEQ ID NO:3 and the second oligonucleotide has a sequence from 15 to 30 contiguous nucleotides, preferably from 18 to 24 contiguous nucleotides chosen in the sequence of SEQ ID NO: 4,
the first oligonucleotide of the second pair of amplification primers has a sequence from 15 to 30 contiguous nucleotides, preferably from 18 to 24 contiguous nucleotides chosen in the sequence of SEQ ID NO: 7 and the second oligonucleotide has a sequence from 15 to 30 contiguous nucleotides, preferably from 18 to 24 contiguous nucleotides chosen in the sequence of SEQ ID NO: 8,
for the detection and/or identification of an alga in a sample likely to contain it, by amplification of at least one variable region of the *LSU gene* of an alga, said variable region constituting a marker of detection and/or identification of an alga, and in which said alga belongs to one of the following phyla: *Glaucophyta, Cyanobacteria, Dinophyta, Ochrophyta, Euglenozoa, Charophyta, Chlorophyta, or Rhodophyta.*

4. Use of a pair of amplification primers according to any one of the preceding claims, wherein said variable region of the LSU gene of an alga is represented by a sequence chosen from the group consisting of SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, or a sequence having an identity percentage of at least 80% with said sequence and in particular 95% and more particularly 98% or 99%.

5. A pair of amplification primers having a size from 15 to 45 nucleotides, in particular from 15 to 30 contiguous nucleotides, preferably from 18 to 24 contiguous nucleotides, wherein a first pair of amplification primers comprises or consists of
- SEQ ID NO: 1, or a sequence having an identity percentage of at least 80%, 81%, 82%, 83%, 84%, 85%, 86%, 86%, 88%, 89%, 90%, 91%, 92%, 93%, 94%, 95%, 96%, 97%, 98%, 99% with said sequence of SEQ ID NO: 1, and
- SEQ ID NO: 2, or a sequence having an identity percentage of at least 80%, 81%, 82%, 83%, 84%, 85%, 86%, 86%, 88%, 89%, 90%, 91%, 92%, 93%, 94%, 95%, 96%, 97%, 98%, 99% with said sequence of SEQ ID NO: 2, wherein a second pair of amplification primers comprises or consists of
- SEQ ID NO: 5, or a sequence having an identity percentage of at least 80%, 81%, 82%, 83%, 84%, 85%, 86%, 86%, 88%, 89%, 90%, 91%, 92%, 93%, 94%, 95%, 96%, 97%, 98%, 99% with said sequence of SEQ ID NO: 5, and
- SEQ ID NO: 6, or a sequence having an identity percentage of at least 80%, 81%, 82%, 83%, 84%, 85%, 86%, 86%, 88%, 89%, 90%, 91%, 92%, 93%, 94%, 95%, 96%, 97%, 98%, 99% with said sequence of SEQ ID NO: 6, allowing to amplify a fragment of a size less than or equal to 220 base pairs of a variable region of the LSU gene coding for the large subunit of a ribosome, of an alga.

6. A pair of amplification primers according to claim 5, said amplification primer having a size from 15 to 30 contiguous nucleotides, preferably from 18 to 24 contiguous nucleotides,
wherein a first pair of amplification primers is chosen in the sequence of SEQ ID NO: 7 and SEQ ID NO: 8 comprising or consisting of a sequence chosen in SEQ ID NO : 20, SEQ ID NO : 21, SEQ ID NO : 22, SEQ ID NO : 23, SEQ ID NO : 24, SEQ ID NO : 25, SEQ ID NO: 26, SEQ ID NO : 27, SEQ ID NO :28, SEQ ID NO : 29, SEQ ID NO : 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO : 39, SEQ ID NO : 40, SEQ ID NO: 41,
wherein a second pair of amplification primers is chosen in the sequence of SEQ ID NO : 3 and SEQ ID NO : 4 comprising or consisting of a sequence chosen in among SEQ ID NO : 42 , SEQ ID NO : 43, SEQ ID NO : 44, SEQ ID NO : 45, SEQ ID NO : 46, SEQ ID NO : 47, SEQ ID NO: 48, SEQ ID NO: 49, SEQ ID NO: 50, SEQ ID NO: 51, SEQ ID NO : 52, SEQ ID NO : 53, SEQ ID NO : 54, SEQ ID NO : 55, SEQ ID NO : 56, SEQ ID NO : 57, SEQ ID NO: 58, SEQ ID NO: 59, SEQ ID NO: 60, SEQ ID NO: 61, SEQ ID NO : 62, SEQ ID NO : 63, SEQ ID NO : 64, SEQ ID NO : 65, SEQ ID NO : 66, SEQ ID NO: 67, SEQ ID NO: 68, SEQ ID NO: 69, SEQ ID NO: 70, or a sequence with an identity percentage of at least 81%, 82%, 83%, 84%, 85%, 86%, 86%, 88%, 89%, 90%, 91%, 92%, 93%, 94%, 95%, 96%, 97%, 98%, 99% or 100% with said sequences.

7. Marker for the detection and/or identification of an alga having a size less than or equal to 220 nucleotides of an amplified variable region of the LSU gene coding for the large subunit of a ribosome, of an alga, in particular less than or equal to 190, 180 and in particular 178 nucleotides, said marker comprising or being constituted by a sequence having an identity percentage of at least 98%, 99%, or 100% with a sequence chosen in, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, and SEQ ID NO: 19.

8. An amplification method for at least one variable region of the *LSU* gene of an alga, said amplified variable region of the LSU gene coding for the large subunit of a ribosome of a size less than or equal to 220 base pairs constituting a marker for the detection and/or identification of an alga, comprising or consisting of the following steps:
(a) a step for the extraction of nucleic material from a product or sample that may contain an alga,
b) a step of amplification of said variable region of the *LSU* gene of an alga, using
- a first pair of amplification primers which consists of a first oligonucleotide which hybridizes to a sequence from 15 to 30 contiguous nucleotides, preferably from 18 to 24 contiguous nucleotides chosen in the SEQ ID NO: 1 sequence of the *LSU gene* of an alga and a second oligonucleotide that hybridizes to a sequence from 15 to 30 contiguous nucleotides, preferably from 18 to 24 contiguous nucleotides chosen in the sequence of SEQ ID NO: 2 of the *LSU gene* of an alga, or
- a second pair of amplification primers which consists of a first oligonucleotide which hybridizes to a sequence from 15 to 30 contiguous nucleotides, preferably from 18 to 24 contiguous nucleotides chosen in the SEQ ID NO:5 sequence of the *LSU gene* of an alga and the second oligonucleotide hybridizes to a sequence from 15 to 30 contiguous nucleotides, preferably from 18 to 24 contiguous nucleotides chosen in the sequence of SEQ ID NO: 6 of the *LSU gene* of an alga,
(c) a step for determining or identifying the presence of an alga in said product or sample by detecting at least one amplified variable region of the *LSU* gene of an alga.

9. The amplification method according to claim 8, wherein said method comprises obtaining at least one amplified variable region of the *LSU* gene of an alga, in particular a step of detecting said obtaining at least one amplified variable region of the *LSU* gene of an alga in order to obtain an amplified and detected variable region of the *LSU* gene of an alga, and wherein said method preferably comprises a step of identification of an alga by sequencing said amplified variable region and detected of the *LSU* gene of an alga in order to obtain a sequenced amplified variable region.
